# EUROPEAN PATENT SPECIFICATION

(11) **EP 4 493 150 B1**
(45) Date of publication and mention of the grant of the patent: **18.11.2026**
(21) Application number: 23710360.1
(22) Date of filing: 14.03.2023
(51) Int. Cl.: A61K 9/00, A61K 9/50

(54) **GRAPHENE-BASED DRUG DELIVERY DEVICE FOR MUCOSAL AND TRANSMUCOSAL ADMINISTRATION**
GRAPHENBASIERTE ARZNEIMITTELABGABEVORRICHTUNG ZUR MUKOSALEN UND TRANSMUKOSALEN VERABREICHUNG
DISPOSITIF D'ADMINISTRATION DE MÉDICAMENT À BASE DE GRAPHÈNE SERVANT À UNE ADMINISTRATION PAR VOIE MUCOSALE ET TRANSMUCOSALE

(30) Priority: 14.03.2022 IT 202200004892
(43) Date of publication of application: 22.01.2025
(73) Proprietor: SISSA - Scuola Internazionale Superiore di Studi Avanzati, 34136 Trieste (TS) (IT)
(72) Inventor: GIUGLIANO, Michele, 34136 Trieste (TS) (IT); BALLERINI, Laura, 34136 Trieste (TS) (IT); FRANCESCHI BIAGIONI, Audrey, 34136 Trieste (TS) (IT)
(74) Representative: Currado, Luisa
(86) International application number: PCT/EP2023/056424
(87) International publication number: WO 2023/174910

(56) References cited:
- CN-A- 108 295 255
- US-A1- 2008 047 926
- WEAVER CASSANDRA L. ET AL: "Electrically Controlled Drug Delivery from Graphene Oxide Nanocomposite Films", ACS NANO, vol. 8, no. 2, 17 January 2014 (2014-01-17), US, pages 1834 - 1843, XP055982457, ISSN: 1936-0851, DOI: 10.1021/nn406223e
- YI JONGDARM ET AL: "Graphene oxide-incorporated hydrogels for biomedical applications", POLYMER JOURNAL, NATURE PUBLISHING GROUP UK, LONDON, vol. 52, no. 8, 8 May 2020 (2020-05-08), pages 823 - 837, XP037193931, ISSN: 0032-3896, [retrieved on 20200508], DOI: 10.1038/S41428-020-0350-9
- SUBRAHMANYAM K. S. ET AL: "Chemical storage of hydrogen in few-layer graphene", PROCEEDINGS OF THE NATIONAL ACADEMY OF SCIENCES, vol. 108, no. 7, 31 January 2011 (2011-01-31), pages 2674 - 2677, XP055983200, ISSN: 0027-8424, DOI: 10.1073/pnas.1019542108

## Description

### FIELD OF THE INVENTION

The present invention relates to the field of drug-delivery systems.

In particular, it relates to a device for mucosal and transmucosal drug delivery providing a controlled release of active pharmaceutical ingredients.

### BACKGROUND OF THE INVENTION

Drug delivery devices with either one-time release, for example for an acute treatment, or long-term operation, for example for chronic treatments, over 2-12 months, are known.

For example US20190091061A1 discloses a drug-delivery system for the treatment of sleep disorders comprising a removable mandibular device with a pharmaceutical compound reservoir for drug release to the oral mucosa upon electrical stimulation.

US7070590B1 discloses a drug-delivery microchip system, based on reservoirs for subcutaneous drug release via an implantable device. This drug-release system is based on passive principles, i.e. drugs diffusion, upon opening the reservoir cap, and active principles, i.e. ion-exchange properties.

Also the use of electrical stimuli was previously proposed to actuate the drug reservoirs, in passive release or ion-responsive drug release systems.

For example, CN102641547 discloses an intraluminal device for transmucosal drug delivery comprising a drug-dispensing portion and an electrically drivable portion which can be operated to dispense the drug from the housing to the area of the mucosal barrier layer that is partially destroyed by the electrically drivable portion.

Similarly, EP2311522 discloses a transmucosal delivery device comprising a drug reservoir and an electrically-actuatable portion.

However, the systems of the prior art do not allow to finely regulate and control the rate, timing and speed of drug release.

Also known in the prior art are reservoirs usually suitable for either one single or several drugs storage. However, in such devices the release actuation cannot be specified independently for each drug, see for example the transmucosal device described in US2014207111.

Most of commercial transmucosal pharmaceutical formulations contain polymer composites that play an important role as a vehicle in the drug-delivery formulations, by providing controlled release of pharmacologic agents. For example in US2021030693 the drug is dispersed on a polymeric film which controls the release of the drug. However, due to its high density the availability of the drug for immediate release to the mucosal membranes progresses slowly.

Weaver et al., 2014, discloses electrically controlled drug delivery from graphene oxide nanocomposite films. The devices do not comprise a microcontroller.

In view of the above, it is still needed a drug delivery system which is finely tunable and useful for both short- and long-term release profiles, suitable for either one single or several drugs administration, with various dosages.

It has now been found a programmable device for transmucosal drug delivery, optionally coupled with a nanoelectrochemical system, for controlled release of active pharmaceutical ingredients which is particularly useful for on-demand drug administration. The present invention takes advantage of the electrical conductivity properties of graphene, and of its potential to operate as a drug- and electron-carrier, to create a programmable drug delivery. These properties provide a miniaturized tunable drug-delivery device, capable of finely regulating rate, timing, and speed of drug release.

The role of graphene as drug carrier for drug delivery system has been explored (see for example WO 2017/066583 A1; CN109464397). It has been also described that graphene-based materials are able to cross physiological barriers (Fadeel et al., 2018 doi:10.1021/acsnano.8b04758).

However, it is known that graphene can elicit organ toxicity effects in the biological system by accumulating in the lung, liver and spleen (Ou et al., 2016, doi:10.1186/s12989-016-0168-y). Therefore, the administration of drugs composition comprising graphene can be dangerous and cause toxic effects in the user.

It has now been found a drug delivery system wherein only drugs are released to mucosalsubmucosa region, while graphene nanoparticles are either kept inside the reservoir or be prevented to reach the mucosa through current-controlled nanomagnetic field that prevents the absorption of the graphene-based magnetic sheets to the biological system, avoiding toxicity.

In particular embodiments, the device of the invention enables extraction of mucosa and submucosa body fluids throughout microneedles controlled by an electromagnetic element for biometric signals measurement based on sensor system.

Biometric signals can be processed by an electronic device, for example by machine learning techniques, to integrate the subjective and interactive evaluation of the user's need of drug delivery device intervention with an objective estimate of the biometric signal processing. The drug delivery device of the invention allows a controlled retainment and release of drugs for extended periods of time, as well as of an immediate release for quick action of drugs at different doses, on an as-needed basis. Therefore, the present innovation allows both immediate-release system and zero-order release system improving pharmacokinetics of mucosa drug release.

### SUMMARY OF THE INVENTION

It has been found a drug delivery system which uses graphene or derivatives thereof selected from the group consisting of graphene oxide, reduced graphene oxide, hydrogenated graphene, graphane, graphone, graphyne, graphdyne, fluorographene as a platform for loading agents via non-covalent interaction and enabling the release of active pharmaceutical ingredients in response to an applied electric potential, at the same time preventing the potential organ toxicity caused by the accumulation of graphene in vital organ when injected systemically. With the device of the invention it is possible to control the speed and dose of the released agent by electrochemical system, indeed application of a low voltage break the pi-interaction resulting in adsorption of the active pharmaceutical ingredient.

The capability of the device of the invention to deliver a drug loaded on graphene or a derivative thereof selected from the group consisting of graphene oxide, reduced graphene oxide, hydrogenated graphene, graphane, graphone, graphyne, graphdiyne, fluorographene and with a modulatory electro-responsive release was demonstrated with caffeine and ibuprofen, however it can be applied to organic and inorganic molecules, polar, apolar, aromatic or non-aromatic pharmaceutical ingredients as well.

It is an object of the present invention, a drug delivery device for mucosal and/or transmucosal drug delivery comprising at least one drug reservoir, said drug reservoir comprising:
- at least one active pharmaceutical ingredient pre-loaded on graphene and/or its derivatives, such as graphene oxide;
- a microcontroller able to deliver electrical current to the inner portion of said drug reservoir and able to connect to an electronic device,

wherein the release of said active pharmaceutical ingredient from said drug reservoir is controlled by said microcontroller and
wherein said drug reservoir comprises a filter on the side of the drug reservoir opposite to the microcontroller, said filter being provided with nanopores allowing the passage of the active pharmaceutical ingredient but not the passage of graphene and/or its derivatives, or
wherein graphene and/or its derivatives are functionalised with magnetic nanoparticles forming graphene-based magnetic nanoparticles and the microcontroller is able to deliver a current-controlled nanomagnetic field which prevents the graphene-based magnetic nanoparticles to reach the mucosa,
wherein graphene derivatives are selected from the group consisting of: graphene oxide, reduced graphene oxide, hydrogenated graphene, graphane, graphone, graphyne, graphdiyne,
fluorographene, optionally modified by covalent or non-covalent functionalization with organic or inorganic molecule.

In an embodiment, the presence of the filter advantageously prevents the graphene and/or derivative thereof to leave the drug reservoir and reach the mucosa.

In another embodiment, graphene and/or its derivatives selected from the group consisting of graphene oxide, reduced graphene oxide, hydrogenated graphene, graphane, graphone, graphyne, graphdiyne, fluorographene are functionalised with magnetic nanoparticles and a current-controlled nanomagnetic field is used, after release of the pharmaceutical ingredient from the complex with graphene, to maintain graphene-based magnetic nanoparticles inside the drug reservoir so that they, advantageously, do not reach the mucosa.

In a preferred embodiment, the pharmaceutical ingredient is loaded on graphene oxide.

In an embodiment, the release of said active pharmaceutical ingredient from graphene and/or its derivatives selected from the group consisting of graphene oxide, reduced graphene oxide, hydrogenated graphene, graphane, graphone, graphyne, graphdiyne, fluorographene occurs upon electrochemical reaction.

In a preferred embodiment, said drug reservoir further comprises an electromagnetic element and said microcontroller further delivers a current-controlled magnetic field through said electromagnetic element.

In a preferred embodiment, said drug reservoir further comprises at least one electrode and said microcontroller delivers electric current through said at least one electrode.

The device of the invention provides the following main advantages:
- Drug release period: a tunable drug-delivery system for both short- and long-term release profiles, even altering drug release schedule in real-time, constantly operating on a time-scale of a few minutes to hours;
- Drug release stimuli: electrical -induced drug-release exploiting graphene properties and inducing an active compound release thanks to its drug- and electron-carrier properties. Electrical stimuli can modulate graphene's ability to establish a balance between physicochemical properties of graphene and bioavailability of drugs. For instance, the amount of drug released at mucosa/submucosa level depend on the graphene electrical conductivity property, allowing on-demand release of drugs be controlled by an electronic unit.
- Pharmaceutical compounds: the drug reservoir is suitable for either one single or several drugs, with various dosage. The properties of graphene as a drug-carrier system offer the peculiar ability to conjugate different drugs in a single graphene sheet, promoting a synergy and versatility of operation controlling additive effects of distinct pharmaceutical compounds.
- Vehicle formulations properties: graphene can immediately deliver the drugs, requiring a reduced amount of polymer or even completely replacing it in the pharmaceutical formulation. Moreover, due to graphene electrical and nanocarriers properties, the electrical currents generated by the microcontroller can induce an electrochemical reaction breaking ionic bonds of the drug-conjugated graphene compound, promptly releasing the active pharmaceutical ingredient and expelling it out of the reservoir, thus enhancing *in situ* availability.

The device of the invention is as defined in the claims.

Embodiments illustrating the invention will be discussed with reference to the following figures.
Figure 1 is a perspective view of a device according to an embodiment of the invention.
Figure 2 is a perspective view of a device according to an embodiment of the invention with a frontal view of a drug reservoir.
Figure 3 is a perspective view of a device according to an embodiment of the invention with a lateral view of a drug reservoir and frontal view of the electrolyte compartment.
Figure 4 is a perspective view of a device according to an embodiment of the invention with lateral views of drug reservoirs.
Figure 5 is a perspective view of a device according to an embodiment of the invention with a frontal view of a sensor reservoir according to an embodiment of the invention.
Figure 6: Current density of caffeine linked to graphene oxide (GO-CAF), graphene oxide-caffeine complex coated with chitosan (CH), ferric sulfate-reduced graphene oxide complex conjugated with caffeine (rGO-F-CAF) and ibuprofen (IBU) linked to GO under electrolysis during 5 minutes.
Figure 7: FTIR spectrum of caffeine released from graphene oxide upon electrochemical reaction. Potential of 1.2 V applied during 5 minutes on (i) caffeine (CAF; as control), (ii) GO conjugated with CAF in presence of saline, (iii) DMSO (as control), (iv) GO conjugated with CAF in presence of dimethyl sulfoxide (DMSO).

### DETAILED DESCRIPTION OF THE INVENTION

Within the meaning of the present invention, for drug delivery device is herein intended a device suitable for the delivery of one or more drugs.

Within the present invention, "active pharmaceutical ingredient" "drug" and "active ingredient" are used as synonyms.

The present invention provides a device for mucosal drug delivery and transmucosal drug delivery. The term "mucosal drug delivery" refers to the release of one or more drugs to any kind of mucosa of a subject for subsequent mucosa absorption. The term "transmucosal drug delivery" refers to the delivery of one or more drugs to any kind of mucosa of a subject that enables the introduction of the active pharmaceutical ingredient at the submucosal level. Said mucosa can be for example oral, nasal or urogenital mucosa.

The device of the invention comprises one or more drug reservoirs. The drug reservoirs can be commercially obtained or manufactured according to methods commonly known in the art, see for example US 20080047926A1. Typically, drug reservoirs are embedded in a matrix able to separate the drug reservoirs one from the other and to provide electrical insulation. The matrix can be commercially obtained or manufactured according to methods commonly known in the art, see for example US 20080047926A1. Preferably, the matrix can be of non-conductive polymers and ceramics, such as epoxy or epoxy resins, silicon dioxide and silicon carbide providing support for the drug reservoirs. The drug reservoirs are positioned in the matrix in such a way to face the mucosa when the device is used in a subject, typically to be in touch with the mucosa. So typically each drug reservoir has at least one side configured to face the mucosa of a subject. The drug reservoir has typically the shape of a cube or of a parallelepiped but other shapes are also suitable. Each side of the drug reservoir may have a length comprised between 10 mm and 50 mm.

The drug reservoir typically comprises a solvent in which the graphene and/or its derivatives selected from the group consisting of graphene oxide, reduced graphene oxide, hydrogenated graphene, graphane, graphone, graphyne, graphdiyne, fluorographene and optionally further compounds are dispersed. For "solvent" it is intended a liquid or gel medium wherein graphene or its derivatives can remain individually dispersed among the solvent molecules. The solvent can be liquid, such as water, physiological saline, ethanol, 1-propanol, ethylene glycol, dimethyl sulfoxide, N,N-dimethylformamide, N-methyl-2-pyrrolidone, tetrahydrofuran and/or gel such as tragacanth, pectin, starch, carbomer, sodium alginate, gelatin, cellulose derivatives, polyvinyl alcohol within a liquid vehicle such as aqueous, hydroalcoholic, alcohol based or non-aqueous.

Each drug reservoir comprises one or more active ingredients loaded on graphene and/or its derivatives selected from the group consisting of graphene oxide, reduced graphene oxide, hydrogenated graphene, graphane, graphone, graphyne, graphdiyne, fluorographene. Graphene derivatives are compounds derived from graphene, which have chemical composition and physical properties similar to graphene, but have been modified or functionalized. Derivatives of graphene include graphene oxide and graphene modified by covalent and non-covalent functionalization.

Graphene and derivatives of graphene selected from the group consisting of graphene oxide, reduced graphene oxide, hydrogenated graphene, graphane, graphone, graphyne, graphdiyne, fluorographene can be modified by covalent and non-covalent functionalization with organic and inorganic molecules, for example with proteins, peptides, fenilamina, polymers or magnetic nanoparticles, as described hereinafter. For example graphene can be covalently functionalized with ferric sulfate.

In a preferred embodiment, the active ingredient is loaded on graphene oxide.

For graphene oxide it is intended a compound of carbon, oxygen, and hydrogen in variable ratios, typically obtained by oxidation and exfoliation of graphite sheets.

Graphene and its derivatives selected from the group consisting of graphene oxide, reduced graphene oxide, hydrogenated graphene, graphane, graphone, graphyne, graphdiyne, fluorographene, such as graphene oxide, can be commercially obtained or manufactured according to methods commonly known in the art (see for example Andrade et al., 2021; Paulchamy et al., 2015).

Preferably, graphene or its derivatives selected from the group consisting of graphene oxide, reduced graphene oxide, hydrogenated graphene, graphane, graphone, graphyne, graphdiyne, fluorographene are in the form of one or more layers sheets, preferably having a thickness of about 1-9 nm with lateral size distribution of about 0.1 µm - 5 mm, for example 0.5 µm - 1 mm.

As mentioned above, graphene and/or its derivatives selected from the group consisting of graphene oxide, reduced graphene oxide, hydrogenated graphene, graphane, graphone, graphyne, graphdiyne, fluorographene can be dispersed in a solvent. Reference can be made for example to Jasim et al., 2016; Liu et al., 2013; Paredes et al., 2008.

The pharmaceutical active ingredient is loaded on graphene and/or its derivatives selected from the group consisting of graphene oxide, reduced graphene oxide, hydrogenated graphene, graphane, graphone, graphyne, graphdiyne, fluorographene according to common methods known in the art, see for example Abdelhamid and Hussein, 2021; Masoudipour et al., 2017. For "loaded on" it is intended that the pharmaceutical ingredient is attached to graphene and/or its derivatives via non-covalent interactions. Typically, the pharmaceutical active ingredient is attached to graphene and/or its derivatives via non-covalent functionalization, see for example Georgakilas et al., 2016.

More than one active pharmaceutical ingredient can be loaded on graphene and/or its derivatives selected from the group consisting of graphene oxide, reduced graphene oxide, hydrogenated graphene, graphane, graphone, graphyne, graphdiyne, fluorographene in the same drug reservoir. For example, two or three active ingredients can be loaded on the same graphene sheet.

In an embodiment, graphene, in particular graphene oxide, after the loading with the desired pharmaceutical ingredient(s) can be coated by one or more polymers such as, but not limited to, polyethylene glycol, poly(pyrrole), poly(ethylene oxide), poly(propylene oxide), poly(vinyl alcohol), polylactic acid, polyacrylic acid, poly lactic-co-glycolic acid and chitosan, thus forming a polymer nanocomposite. Polymer nanocomposite comprising graphene coated by a polymer can be obtained according to common methods known in the art, see for example Singh et al., 2012. For polymer nanocomposite it is intended a polymer or copolymer as a composite material comprising a polymer matrix having graphene and/or its derivatives as a dispersed nanofiller appropriately distributed in the polymer matrix wherein at least one dimension of the matrix is in the range of 1-100 nm (see for example Lawal et al., 2020). The presence of the polymer advantageously provides additional stability for the drug to be released and can also function as a vehicle for the drug, as described in Yi et al., 2020. The polymer nanocomposites can or can not be released from the reservoir together with the active ingredient.

Coating of the graphene with the polymer occurs as known in the art see for example Ahmad et al., 2019; Pei et al., 2020, for example by way of dispersion methods producing noncovalent functionalized composites.

In some embodiments, graphene sheets are functionalised with one or more magnetic nanoparticles comprising for example Fe₃O₄ (magnetite), γ-Fe₂O₃ (maghemite), magnetic chitosan composites and Fe₃O₄/SiO₂ (magnetite-silica coated surface). Coating of graphene with magnetic nanoparticles occurs before graphene is loaded with the desired pharmaceutical ingredient(s).

Coating of graphene with magnetic nanoparticles occurs as known in the art, typically it is produced by way of covalent synthesis methods, see for example Huang et al., 2020; Yang et al., 2015. Graphene-based magnetic nanoparticles may provide large surface area for functionalization, magnetism and high carrier mobility for delivering macromolecules active pharmaceutical ingredients, which are generally not well absorbed through the mucosa, across the mucosa membrane.

The drug reservoir can further comprise one or more compounds useful for improving drug formulation preparation and mucosal permeability as described in the art (Sharma et al., 2006; Davis and Illum, 2003). Such compounds can be one or more of excipients, surfactants, preservatives, stabilizers, chelating agents, enzyme inhibitors, antibacterial agents such as, sodium taurodihydrofusidate, sugar, sodium glycocholate, lecithins, polycarbophil, didecanoyl-l-alpha-phosphatidylcholine, dioctylsulphosuccinate, methylcellulose, chitosan and chitosan derivative, cyclodextrins, polysorbate-80, polyethyleneglycol-8-laurate, glyceryl monolaurate, laurenth-9, lysophosphatidyl choline and palmitoylcamitine chloride, benzalkonium chloride. Such further compound can be included in the solvent. If two or more further compounds are present, they can be included in the solvent as a homogeneous mixture.

In an embodiment, such compounds useful for improving drug formulation preparation and mucosal permeability can be included in a compartment inside the drug reservoir.

The drug reservoir further comprises a microcontroller which is usually at one end of the drug reservoir, preferably at the end opposite to the portion of the drug reservoir which is configured to face the mucosa. The microcontroller is able to deliver an electrical current and/or a current-controlled magnetic field to the inner portion of the drug reservoir, containing the graphene loaded with the active ingredient.

In a preferred embodiment, the microcontroller delivers electrical current to the inner portion of the drug reservoir through at least one electrode, preferably two electrodes, preferably located on opposite walls of the drug reservoir.

In another preferred embodiment, the microcontroller delivers a current-controlled magnetic field to the inner portion of the drug reservoir through an electromagnetic element. Such electromagnetic element can be a solenoid, toroid, electromagnet or electromagnetic coil, preferably it is a solenoid.

The microcontroller is also able to connect via radio signals, e.g. near field communication, radio-frequency identification and wireless network interconnecting via wireless personal area network or local area network, with an electronic device such as a smart-phone, a tablet or a personal computer as described by Farra et al., 2012. The user can modulate from the electronic device the kind and timing of deliver of electrical stimulus released from the microcontroller and consequently control the drug release. Advantageously, the user can thus easily configure the timing and rate of drug release from the electronic device and the drug can be released in a programmable on-demand fashion. The electronic device typically has an interface allowing the user to control and schedule the desired drug administration.

In an embodiment, the microcontroller is connected to two electrodes (an anode and a cathode), enabling the delivery of an electrical current upon wireless signal by a connected electronic device. The electrodes can be commercially obtained or manufactured according to methods commonly known in the art (Guo et al., 2016). Preferably, the electrodes are placed on opposite walls of the reservoir. The potential applied by the microcontroller is used to induce electrolysis, which determines the release of the pharmaceutical ingredient(s) from the graphene and its derivatives by desorption. Such electrolysis may occur in presence of electrolytes.

In an embodiment, the device further comprises one or more electrolyte compartment embedded in the same matrix in which the drug reservoirs are embedded. Electrolyte compartment may be filled with electrolyte solution, which preferably comprises one or more of physiological sodium chloride, dimethyl sulfoxide, ringer's solution, lactated ringer's solution, phosphate-buffered saline, Hank's balanced salt solution, glycerol, ethanol or poly (ethylene glycol)-grafted chitosan.

Electrolyte compartment may be placed adjacent to the drug reservoir and preferably separated from it by a stimuli-responsive polymeric wall. Said stimuli-responsive polymer wall can comprise a polymer such as polypyrrole, poly(3,4-ethylenedioxythiophene), poly(N-isopropylacrylamide), poly(lactic-co-glycolic acid), polyethylene glycol, chitosan/Fe3O4, polypyrrole-chitosan or xanthan/Fe3O4.

In an embodiment, the electrolyte compartment further comprises a cathode which, when receiving a signal from the microcontroller, delivers electrical stimulus to said stimuli-responsive polymer wall. In this embodiment, said wall functions as an anode and under electrical stimulus can disintegrate or modify its structure in the form of porous, allowing the electrolytes pass through the polymer wall reaching inside the reservoir. Advantageously, once inside the reservoir, such electrolyte may facilitate the flow of charge between the two reservoir electrodes, allowing the electrochemical reaction to proceed. Electrochemical reaction is defined as electrolysis process, at which an external source of electrical current is used to drive a non-spontaneous redox reaction. The electrochemical reaction is able to break the bonds between the drug-conjugated graphene compound present in the drug reservoir, promptly releasing the active pharmaceutical ingredients from graphene and its derivatives. In one embodiment the electrolyte may facilitate the production of hydrogen gas inside the reservoir, which may affect the electrostatic, hydrophobic and π-π interactions between active pharmaceutical ingredients and graphene and its derivatives allowing the releasing process. Moreover, the hydrogen gas formation may lead to an increasing of the pressure inside the reservoir, advantageously allowing a quick extravasation of the pharmaceutical ingredients out of the reservoir.

In an embodiment, the drug reservoir further comprises an excipient compartment placed adjacent to the compartment comprising the active pharmaceutical ingredient loaded on graphene, on the side opposite to the microcontroller, and separated by the compartment comprising the active pharmaceutical ingredient loaded on graphene by a nanofilter. Said further compartment comprises one or more excipients that may enhance the bioavailability of the active pharmaceutical ingredients. The excipient can be chosen among the excipients known in the pharmaceutical field.

For microcontroller is intended a small computer on a single VLSI integrated circuit (IC) chip. In an embodiment, the microcontroller is or comprises a microprocessor.

In a preferred embodiment, the microcontroller is a battery-operated miniaturized digital embedded electronic system. Battery is preferably rechargeable by wireless power transfer, but it may be disposable. The microcontroller can comprise battery-charge regulation and control, low-power wireless Bluetooth data transfer, and analog real-time operation as one- or two-channel voltage-controlled or current-controlled stimulus isolator. Such microcontroller is commercially available as standard off-the-shelf microcontroller or can be manufactured according to common general knowledge in the field, see for example Abadal et al., 2013; Furniturewalla et al., 2018; Kang et al., 2021; Lukas et al., 2019. The microcontroller can generate repeated biphasic voltage or current pulses, for example with amplitudes in the range of 0 - 5V, which are able to reach the graphene sheets. Repetition rates are preferably programmable, typically in the range 0-50 Hz, allowing advantageously to determine the desired drug-delivery concentration profile.

The drug reservoir can further comprise one or more electromagnetic device, such as a solenoid, able to deliver a magnetic field upon electrical control. In an embodiment said electromagnetic device is located inside the drug reservoir, preferably on the opposite side with respect to the microcontroller. In another embodiment said electromagnetic device surrounds the drug reservoir. Such electromagnetic device can be connected to the microcontroller from which it may receive signals. For example one or more electromagnetic solenoid can be present on the opposite side of the microcontroller. In an embodiment, the microcontroller is connected to an electromagnetic solenoid through a relay module, which is an electromechanical switch used to turn the solenoid on or off. It typically consists of a coil and a set of contacts. The electromagnetic solenoid can be commercially obtained or manufactured according to methods commonly known in the art (Khalifa et al., 2021).

The invention will be now further described with reference to the enclosed figures.

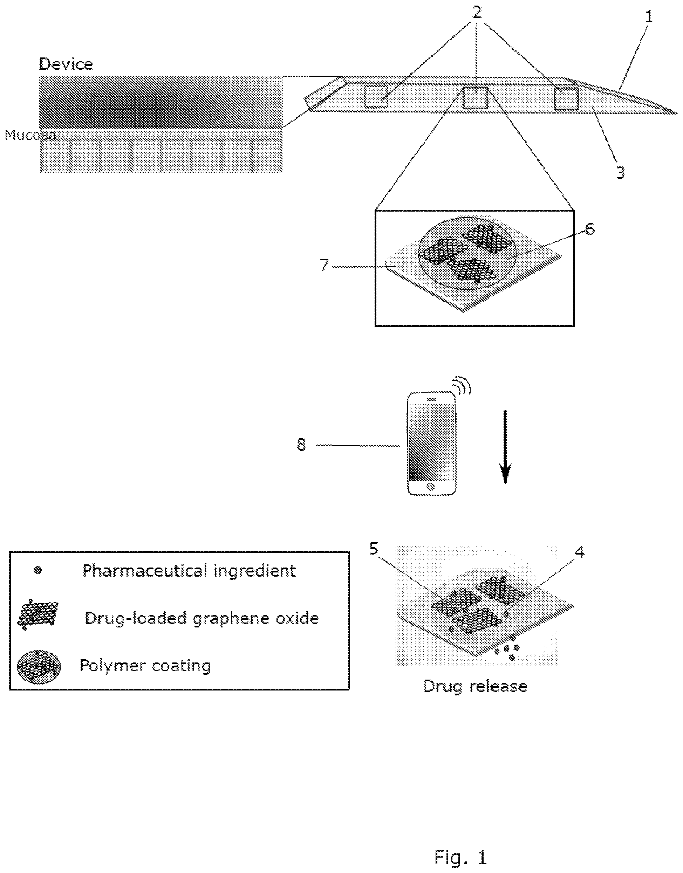

With reference to figure 1 the device of the invention 1 comprises drug reservoirs 2, embedded in a matrix 3. Each drug reservoir comprises active ingredient(s) 4 loaded on graphene oxide sheets 5 and coated by a polymer, this forming a polymer nanocomposite 6. A microcontroller 7 is present at the top of the reservoir.

In an example of functioning of the device of the invention, the microcontroller 7 receives a signal from an electronic device, e.g. a smartphone 8, to deliver electrical current.

The electrical current determines the release of the pharmaceutical ingredient(s) conjugated to the graphene oxide from the drug reservoir. Intensity and timing of the electrical stimuli determine different modalities of release of the drug and they can be operated by a user through an electronical device connected to the microcontroller. With regard to the modulation of the drug release through electrical stimulation reference can be made to the work of Weaver et al., 2014. For example electrically controlled release of active pharmaceutical ingredients can be achieved under an applied voltage of -0.5 V for 5 s followed by 0.5 V for 5 s delivered by the microcontroller (see Wu et al., 2015; Reddy et al., 2018; Weaver et al. 2014, Hou et al., 2020). Such electrical stimulus may affect the electrostatic, hydrophobic and π-π interactions between active pharmaceutical ingredients and graphene oxide and its derivatives allowing the releasing process, see for example Weaver 2014.

The electronical device may be orchestrated by an application software upon a command signal to generate a voltage impulse delivered by the microcontroller.

Application software can be based on responsive web design as known in the art (Weichbroth, 2020), e.g. follow IEEE guidelines for mobile app design. The application software can display information about the operating system such as controller of dose or programming the time period of the drug to be delivery. Application software may also be used for monitoring the drug delivery device status such as amount of drug in the reservoir, a log of previous drug deliveries including the amount and time of drug delivered.

The microcontroller may also generate a signal to be transmitted to the electronic device after the drug delivery operation to confirm the appropriated delivery of the drug. Information can be transmitted by the electronic device to other electronic devices upon an appropriate command.

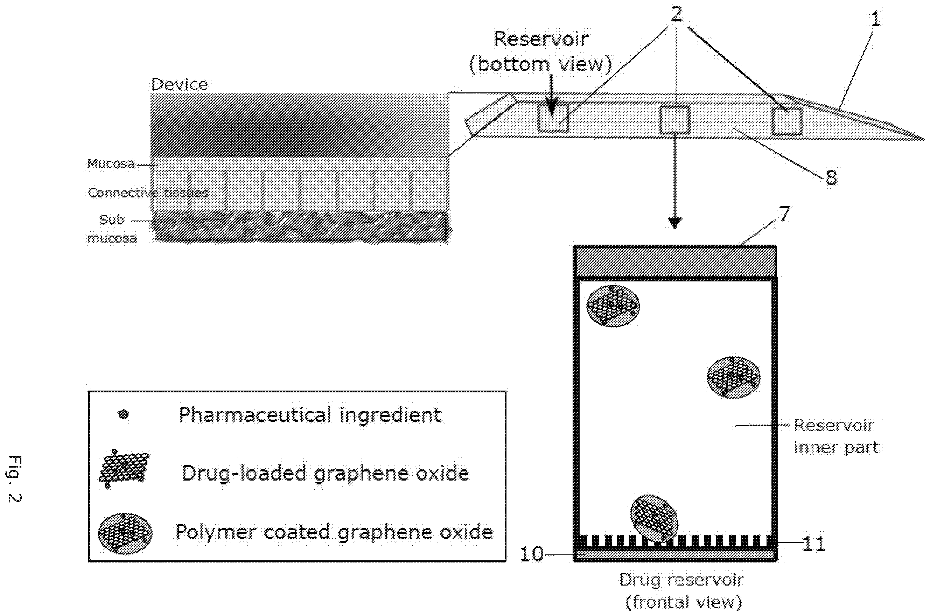

With reference to figure 2, the device of the invention 1 comprises drug reservoirs 2 embedded in a matrix 3. The drug reservoir is provided with a microcontroller 7 able to deliver electrical current to the inner portion of said drug reservoir and able to connect to an electronic device. The drug reservoir is provided with a reservoir cap 10 located at the end of the drug reservoir which is configured to face the mucosa. The reservoir cap prevents the leak of the active ingredient from the drug reservoir. Reservoir cap preferably comprises stimuli-responsive polymer composites such as polypyrrole, poly(3,4-ethylenedioxythiophene), polyvinyl alcohol, polyethylene glycol, poly(lactic-co-glycolic acid), polydopamine, chitosan/Fe₃O₄ or xanthan/Fe₃O₄ combined with one or more metals such as gold, silver, and zinc. For stimuli-responsive polymer composite is intended a material that can change its structure and properties in the presence of a physical stimulus. The drug reservoir also comprises a nano filter 11 with nanopores allowing the passage of pharmaceutical ingredients but not the passage of graphene sheets, for example pores having diameter in the range of 5-500 nm, for example 5-400 nm, preferably 250-450 nm. The nano filter advantageously permits the exclusive passage of the pharmaceutical active ingredient so that the graphene oxide does not reach the mucosa.

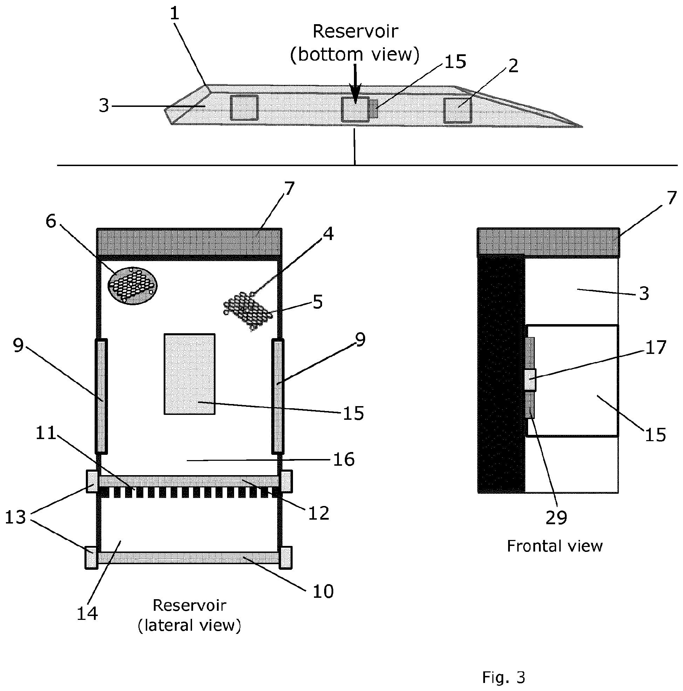

With reference to figure 3, the device of the invention 1 comprises drug reservoirs 2 embedded in a matrix 3. The drug reservoir comprises pharmaceutical ingredient 4 conjugated to graphene and/or its derivatives 5. The drug reservoir is provided with two electrodes 9 (an anode and a cathode) placed in the opposite walls of the reservoir and connected to the microcontroller, allowing the electrical stimulus to be applied. The potential applied by the microcontroller is used to induce electrolysis, which determines the release of the pharmaceutical ingredient(s) from the graphene and its derivatives by desorption. The drug reservoir also comprises a reservoir cap 10 located at the end of the drug reservoir which is configured to face the mucosa. Two electrodes 13, an anode and a cathode, are placed on opposite walls of the reservoirs in contact with the reservoir cap. The reservoir cap works as the anode where the oxidation reaction occurs, causing disintegration by electrical, or magnetic field stimulus delivered by the microcontroller through the cathode pole, or through the electromagnetic solenoid, respectively, allowing the release of the pharmaceutical ingredients from the reservoir, as described by Kwon et al., 1990. Cathode is preferably made of metal or non-metal such as copper, gold, silver, platinum, carbon, carbide or graphite.

The reservoir also comprises a filter cover 12 placed on top of the nanofilter 11 which separates the reservoir content from the filter. Such filter cover may be preferably made of a stimuli-responsive polymer such as polypyrrole, poly(3,4-ethylenedioxythiophene), poly(N-isopropylacrylamide), poly(lactic-co-glycolic acid), polyethylene glycol, chitosan/Fe3O4, polypyrrole-chitosan or xanthan/Fe3O4 optionally combined with one or more metals such as copper, gold, silver, and zinc.

On both sides of the filter cover electrodes 13 are present. When an electrical current is applied from the microcontroller the electrode 13 functions as cathode and the filter cover 12 functions as anode where the oxidation reaction occurs, thus advantageously allowing to control the time of filtering of the reservoir content. The electrode preferably comprises a metal or non-metal material, such as copper, gold, silver, platinum, carbon or graphite.

The drug reservoir also comprises an excipient compartment 14 placed adjacent to the filter and comprising one or more excipients that may enhance the bioavailability of the active pharmaceutical ingredients.

The device of the invention 1 also comprises an electrolyte compartment 15 embedded in the matrix 3. Electrolyte compartment may be filled with electrolyte solution, which preferably comprises one or more of physiological sodium chloride, dimethyl sulfoxide, ringer's solution, lactated ringer's solution, phosphate-buffered saline, Hank's balanced salt solution. Electrolyte compartment may be separated from the drug reservoir by a stimuli-responsive polymer wall 29, made of for example of polypyrrole, poly(3,4-ethylenedioxythiophene), poly(N-isopropylacrylamide), poly(lactic-co-glycolic acid), polyethylene glycol, chitosan/Fe3O4, polypyrrole-chitosan or xanthan/Fe3O4. Electrolyte compartment may also comprise of cathode 17 to delivery electrical stimulus to the stimuli-responsive polymer wall, which under electrical stimulus leads to a disintegration or porous formation, allowing the electrolytes pass through the polymer wall reaching inside the reservoir. Such electrolytes may facilitate the flow of charge between the two reservoir electrodes, allowing the electrochemical reaction to proceed. Electrochemical reaction is defined as electrolysis process, at which an external source of electrical current is used to drive a non-spontaneous redox reaction. This process can be used to extract pharmaceutical ingredients from graphene and its derivatives or produce hydrogen gas, which may affect the electrostatic, hydrophobic and π-π interactions between active pharmaceutical ingredients and graphene and its derivatives allowing the releasing process.

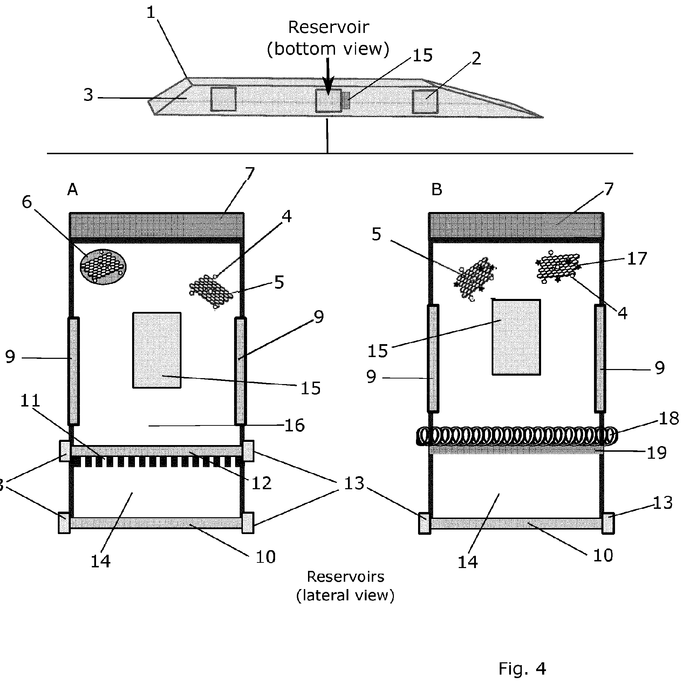

In figure 4 two embodiments of the drug reservoir and of mechanisms of release of the active ingredient from the reservoir are shown.

The device of the invention 1 comprises several drug reservoirs 2 embedded in a matrix 3.

In the drug reservoir on the left (A) the microcontroller 7, controlled by an electronic device, delivers an electrical stimulus to the stimuli-responsive polymer wall of the electrolyte compartment 15, which under electrical stimulus leads to its disintegration or porous formation, allowing the electrolytes pass through the polymer wall and reaching inside the reservoir 16. Such electrolytes may facilitate the flow of charge between the two reservoir electrodes 9, allowing the electrolysis process. The microcontroller also delivers an electrical stimulus inside the drug reservoir 16 through two electrodes 9, an anode and a cathode, placed on opposite walls of reservoir 2. When the electrical stimulus is applied to the electrode the electrolysis starts. Electrodes are preferably made of metals such as copper, gold, silver, zinc, platinum, carbon or graphite.

Such electrolysis is an electrochemical reaction defined as an electric current that is sent through the electrolytes and into the dispersion of graphene 5 conjugated with pharmaceutical ingredients 4 in order to stimulate the flow of ions necessary to separate pharmaceutical ingredients and graphene and its derivatives sheets. During electrolysis, an oxidation-reduction reaction takes place at the electrodes as a result of the flow of electric current through the electrolyte solution. In some embodiments, electrolysis may also be used to produce hydrogen gas at the anode, which may affect the electrostatic, hydrophobic and π-π interactions between active pharmaceutical ingredients and graphene and its derivatives allowing the releasing process by desorption. Following such electrochemical reaction, the active ingredient 4 is released from graphene oxide 5. The polymer coating 6 can be released together with the active ingredient. A nano filter 11 is present at the end of the drug reservoir opposite to the microcontroller 7 allowing the exclusive release of the active ingredient. The reservoir also comprise a filter cover 12 placed on top of the filter, separating the reservoir content from the filter to control the time of filtering of the reservoir content. On both sides of the filter cover electrodes 13 are present. When an electrical current is applied from the microcontroller the electrode 13 functions as cathode and the filter cover 12 functions as anode where the oxidation reaction occurs, thus advantageously allowing to control the time of filtering of the reservoir content.

The reservoir also comprises an excipient compartment 14 placed adjacent to the filter to store excipients that may enhance the bioavailability of the active pharmaceutical ingredients.

Reservoir comprises a reservoir cap 10 located at the end of the drug reservoir which is configured to face the mucosa. Two electrodes 13, an anode and a cathode, are placed on opposite walls of the reservoir in contact with the reservoir cap. Electrical current can be delivered from these electrodes to the reservoir cup determining its disintegration, therefore allowing to control the release of the reservoir content and of the excipients to the mucosa.

**In** the drug reservoir on the right (B), graphene oxide sheets and its derivatives 5 loaded with the pharmaceutical ingredient 4 are conjugated with magnetic nanoparticles 17. The microcontroller 7, controlled by an electronic device, delivers an electrical stimulus inside the drug reservoir through the electrodes 9, an anode and a cathode, placed in the opposite walls of reservoir. When the electrical stimulus is applied to the electrode the electrochemical reaction starts. An electromagnet solenoid 18 is present on the side of the reservoir opposite to the microcontroller and connected to said microcontroller. The reservoir also comprises a gate 19 placed adjacent to the solenoid 18 and controlled by it allowing the opening and closing of the reservoir. Gate can be preferably of non-conductive polymers and ceramics, polyethylene, polypropylene, polystyrene, polyvinyl chloride, poly(methyl methacrylate), polyethylene terephthalate, polycarbonate, silicon dioxide and silicon carbide.

Reservoir also comprises a reservoir cap 10 located at the end of the drug reservoir which is configured to face the mucosa, controlling the time that reservoir content, and excipients are delivered to the mucosa thanks to the electrical stimulus received by the two electrodes 13, an anode and a cathode, placed on opposite walls of the reservoirs in contact with the reservoir cap.

**In** an example of functioning of the device, a first electric stimulus is operated by the microcontroller in order to deliver an electrical current to the stimuli-responsive polymer wall of the electrolyte compartment 15 through the cathode 9, which under electrical stimulus leads to a disintegration or porous formation of the wall, allowing the electrolytes pass through the polymer wall reaching inside the reservoir. A second electric stimulus is operated by the microcontroller inside the drug reservoir through the cathode 9 in order to induce the electrolysis process, which allows the release of the pharmaceutical ingredients from magnetic graphene nanoparticles. Subsequently, the electromagnet solenoid 18 is operated to attract the magnetic graphene nanoparticles, consequently preventing the release of the graphene-based magnetic sheets from the reservoir. When an electrical current passes through the electromagnetic solenoid, it generates a magnetic field, which exerts a force of attraction, pulling the magnetic graphene nanoparticles towards the solenoid. The solenoid frame is then operated by the microcontroller to activate the gate 19 by electrical stimulus, opening the reservoir and allowing only the pharmaceutical ingredients to reach the excipient compartment 14. After the pharmaceutical active ingredients have reached the excipient compartment, the reservoir gate is operated by the solenoid frame to close the reservoir, preventing the releasing of graphene sheets. Then, the electromagnet solenoid can be switched off. Finally, the reservoir cap can be disintegrated by electrical stimulus delivered by the microcontroller through the cathode pole 13, allowing the release of the pharmaceutical ingredients and excipients from the reservoir. Such embodiment of the reservoir is suitable for magnetic nanoparticles conjugated with graphene or its derivatives and loaded with pharmaceutical ingredient.

In an embodiment, the device of the invention comprises one or more sensor reservoirs. Each sensor reservoir comprises a first compartment comprising at least one microneedle configured to puncture the mucosa, and a second compartment separated from said first compartment by a matrix and comprising a microcontroller, an electromagnetic element connected to said microcontroller, at least one microplunger connected to said microneedle and operated by said electromagnetic element and an electrochemical biosensor. The electromagnetic element, which is preferably a solenoid frame, pulls the microneedles toward the mucosa through operation of the microplunger thus allowing the microneedles to reach the biological tissue and collect the body fluid. The solenoid then push the microplunger up so that the body fluid can reach the second compartment and the electrochemical biosensor. Preferably, more than one microneedle is present and they are typically arranged in an array. The body fluid can be or comprise mucosa body fluid, submucosa body fluid, epithelial mucosa, fluid mucosal, blood, saliva, seminal fluid, vaginal fluids, mucus and/or phlegm.

An electrochemical biosensor typically comprises a receptor for the biological element to be recognized, an electrochemical transducer that converts the biochemical reaction induced by the binding of the biological element in a measurable signal and a signal processing unit which provides the interpretation of the signal. Electrochemical biosensors, herein named also "biochemical sensors", are of common use in the field and commercially available. The biological element recognized can be epithelial mucosa, fluid mucosal, blood, saliva, seminal fluid, vaginal fluids, mucus and phlegm. Electrochemical biosensors can provide information regarding the presence and quantity of an analyte in the biological element, such as gases, organic compounds, local cells, hormones, drug or its metabolites, enzymes, bacterial and viral pathogens, temperature, pH. Reference can be made for example to Kim et al., 2015; Slaughter and Kulkarni, 2017; Stevenson et al., 2019. Depending on the body fluid to be extracted, one or more suitable electrochemical biosensors may be used according to the general knowledge in the field.

The signals obtained by the biochemical sensors can be transmitted to an electronic device, such as a personal computer, a tablet or a smartphone. Said electronic device can be also connected with the microcontrollers embedded in the drug reservoirs.

The microcontroller has the same features described above in relation to the drug reservoir, in particular it is able to emit electrical stimulus.

The sensor reservoir is configured to be in contact with the mucosa when the device is used in a subject.

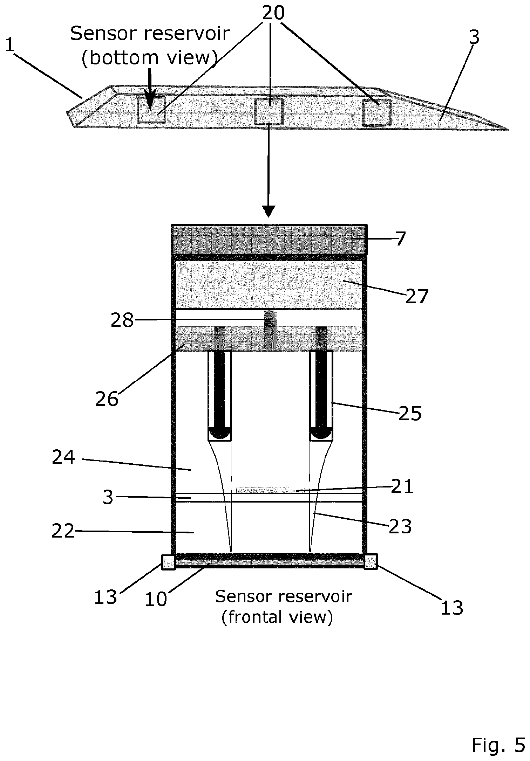

A particular embodiment of a sensor reservoir is herein described with reference to figure 5. **In** figure 5 the drug delivery device of the invention 1 comprises sensor reservoirs 20, embedded in the matrix 3. Each sensor reservoir comprises an electrochemical biosensor 21 and a microcontroller 7 located on the end of the reservoir opposite to the portion which is configured to face the mucosa. The sensor reservoir comprises a first compartment 22 located at the end of the reservoir configured to face the mucosa sealed with a reservoir cap 10 flanked by two electrodes 13. Said first compartment comprises microneedles 23 configured to puncture the mucosa. The sensor reservoir comprises a second compartment 24, separated from said first compartment 22 by the matrix 3. Said second compartment comprises the biochemical sensors 21 and microplungers 25 connected on one side to a plunger head 26 and on the opposite side to the microneedles 23. The second compartment 24 also comprises a solenoid frame 27 positioned between the microcontroller 7 and the plunger head 26 and connected to said plunger head by a solenoid plunger 28. The electromagnet solenoid can be commercially obtained or manufactured according to methods commonly known in the art (Song and Lee, 2015). The plunger head can be preferably of non-conductive polymers and ceramics, polyethylene, polypropylene, polystyrene, polyvinyl chloride, poly(methyl methacrylate), polyethylene terephthalate, polycarbonate, silicon dioxide and silicon carbide.

**In** an example of functioning of the sensor reservoir of the invention, the microcontroller delivers an electrical current to the solenoid frame to operate the solenoid plunger. The solenoid frame moves the solenoid plunger down, pulling the microneedle toward the mucosa thus allowing the microneedle to reach the biological tissue to collect the body fluid. The plunger is then moved up by the solenoid to suck the body fluid which can then reach the electrochemical biosensor. The microcontroller 7 is able to connect with an electronic device through which the emission of electrical stimulus and consequently the movement of the microneedles are controlled.

Another object of the invention is an integrated system comprising:
- a drug delivery device as herein described able to deliver one or more drugs to the mucosa of a subject;
- a biosensor system able to collect biometric information from said subject;
- an electronic device able to connect to both said drug delivery device and said biosensor system,
wherein the delivery of the drug from said drug delivery device is controlled by said electronic device based on the information received from said biosensor system.

The biosensor system can be a device suitable to be applied to the mucosa of a subject comprising one or more sensor reservoirs as above described.

In an embodiment, the biosensor system is located on said drug delivery device.

In other embodiments, the biosensor system can comprise one or more sensors suitable to be applied to a subject and to collect biometric information.

For example biometric information from the subject can be collected by remote sensors placed on the body of the user, such as wearable sensor patches or devices, which detect biometric information and send them to an electronic device. Remote sensors collecting biometric information are commercially available. Wearable devices can be for example commercially available smart-watches or fitness bracelets.

Biometric information can comprise skin conductivity, heart rate, blood oxygenation.

The electronic device, e.g. a personal computer, a smart-phone or a tablet, processes the information received from the biosensor system, located inside the drug delivery device or as a separate system, and optionally further information inserted by a user to provide an estimate of the condition of the subject, for example by an application software. Information processing can advantageously be carried out by machine learning techniques, which are well known in the field. The estimation of the condition of the subject allows to monitor in real time the physiological response of the subject to the drug-delivery. The electronic device can thus combine the feedback obtained by the biosensor system with the operation of the drug delivery device in a closed-loop process advantageously monitoring in real time the effects of the drug delivery and decreasing the chance for accidental overdosage of the compounds.

The drug delivery device of the invention can be advantageously used for the treatment of any condition which would take advantage from a transmucosal administration of a pharmaceutical ingredient. For example, it can be used for the treatment of gastroenterology, hematology, infectious, neurology, psychiatry, endocrinology, nutrition, genitourinary and respiratory tract diseases, in particular for the treatment of at least one disorder or condition selected from the following group: gastrointestinal disorders, genitourinary disorders, psychiatric disorder, neurological disorder and nutritional deficiency, including but not limited to adrenal insufficiency, allergy, anxiety disorder, asthma, attention-deficit hyperactivity disorder, bipolar disorder, chronic and acute pain, chronic bronchitis, chronic obstructive pulmonary disease, chronic obstructive pulmonary disease, congestion, cramp, cyclic vomiting syndrome, dementias, dependence syndrome, diabetes mellitus, epilepsy, fever, genitourinary disorder, hypercholesterolemia, hormonal imbalance, hyperglycemia, hypersalivation, hypertension, hypotension, hypothyroidism, infectious disease, inflammatory diseases, menopause, menstrual regulation, migraine, mood disorders, musculoskeletal pain, nausea, neoplasms, obesity, Parkinson's disease, pulmonary disease, respiratory disease, sexual dysfunction, sleep disorder, smoking cessation, vaginal diseases, vomiting.

The pharmaceutical ingredient to be delivered can be selected from the skilled person depending on the pathology to be treated and the conditions of the patients. In an embodiment, the pharmaceutical ingredient is for the treatment of at least one disorder or condition selected from the following group: gastrointestinal disorders, genitourinary disorders, psychiatric disorder, neurological disorder and nutritional deficiency, including but not limited to adrenal insufficiency, allergy, anxiety disorder, asthma, attention-deficit hyperactivity disorder, bipolar disorder, chronic and acute pain, chronic bronchitis, chronic obstructive pulmonary disease, chronic obstructive pulmonary disease, congestion, cramp, cyclic vomiting syndrome, dementias, dependence syndrome, diabetes mellitus, epilepsy, fever, genitourinary disorder, hypercholesterolemia, hormonal imbalance, hyperglycemia, hypersalivation, hypertension, hypotension, hypothyroidism, infectious disease, inflammatory diseases, menopause, menstrual regulation, migraine, mood disorders, musculoskeletal pain, nausea, neoplasms, obesity, Parkinson's disease, pulmonary disease, respiratory disease, sexual dysfunction, sleep disorder, smoking cessation, vaginal diseases, vomiting.

In an embodiment, the pharmaceutical ingredient is one of the followings: analgesics, anesthetics, angiotensin-converting enzyme agent, anorectic, anti-addiction agents, antiallergy agent, antibacterials, anticonvulsants, anxiolytics, antidementia agents, antidepressants, antidiabetic agent, anti-diuretic, antiemetics, antiepileptic, antifungals, antihelminthic, antihistamine, anti-hyperglycemic, antihypertensive agent, anti-hypotensive agent, antiinflammatories, antimigraine agents, antimuscarinic, antineoplastics, anti-obesity agents, antiparasitics, anti-Parkinson agent, antipyretics, antipsychotic, antispasmodic, antiviral, anxiolytic, benzodiazepines, beta adrenergic blocking agents, bronchodilator, cardiovascular agent, central nervous system agents, cholesterol lowering drug, contraceptives, cough suppressant, dopaminergic agonist, expectorant, gastrointestinal agent, genitourinary agents, glycogenolytic agents, hormonal agents, immunosuppressant agent, minerals, mucolytic, muscle relaxant, nasal decongestant, neurosteroids, opioid agonist, parasympathomimetic, pherine, prostaglandin, respiratory tract agents, sedative, sexual disorder agents, smoking cessation agents, sleep disorder agents, stimulant, vitamins.

In particular, the pharmaceutical ingredient can be selected from the group consisting of: acyclovir, albuterol, alclometasone, alfentanil, allergen extracts, allopregnanolone, almotriptan, aloradine, alprazolam, alprostadil, amoxicillin, amphetamines, androstanediol, apomorphine, arctigenin, articaine, ascorbic acid, asenapine, atomoxetine, atorvastatin, atropine, azathioprine, azelastine, barbiturate, beclometasone, bisphosphonates, budesonide, bupivacaine, buprenorphine, bupropion, caffeine, cannabidiol, captopril, carfentanil, chlordiazepoxide, chloropheniramine, chlorpromazine, cholecalciferol, clonidine, clorazepate, clorazepic acid, cobalamin, cortisone, cromolyn, cyclobenzaprine, desmopressin, dexamethasone, dextroamphetamine, diazepam, diethylcarbamazine, dihydroergotamine, dinoprostone, donepezil, econazole, eletriptan, endorphins, enkephalins, ephedrine, epinephrine, ergoloid, ergonovine, ergotamine, estazolam, estradiol, estropipate, etonogestrel, fentanyl, fluconazole, fludrocortisone, fluoxetine, flurazepam, fluticasone, fluticasone, framycetin, frovatriptan, furosemide, gabapentin, ganaxolone, gentamicin, glimepiride, glucagon, gramicidin, guaifenesin, halazepam, heparin, hydrocortisone, hyoscyamine, ibuprofen, insulin, ipratropium, isoproterenol, ivermectin, ketamine, ketoprofen, labetalol, levothyroxine, lidocaine, lofentanil, loratadine, lorazepam, magnesium, melatonin, mepivacaine, metformin, methadone, methylphenidate, methylsergide, methyltestosterone, metoclopramide, metoprolol, metronidazole, midazolam, midazolam, misoprostol, mometasone, morphine, nadolol, nalbuphine, naloxone, naltrexone, naproxen, naratriptan, nicotine, nifedipine, nimesulide, nitroglycerin, norethindrone, novocaine, olanzapine, ondansetron, opiates, oxycodone, oxycodone, oxymetazoline, oxymetazoline, oxytocic, paracetamol, pentobarbital, phenylephrine, phytonadione, pilocarpine, prazosin, prednisone, pregabalin, pregnanolone, prilocaine, prilocaine, promethazine, propofol, propranolol, prostaglandin, pyridoxine, retinol, rizaptril, salicylic acid, salmeterol, scopolamine, segesterone, selegiline, sildenafil, sufentanil, sumatriptan, temazepam, terbutaline, tetracaine, thiamine, thymosin, timolol, tinidazole, tocopherol, tramadol, triamcinolone, triazolam, tryptophan, vardenafil, varenicline, verapamil, zoledronic acid, zolmitriptan, zolpidem. Preferred active pharmaceutical ingredients are caffeine and ibuprofen.

In particular, the pharmaceutical ingredient and respective disorder or condition to be treated, can be as follows: acetylsalicylic acid for anticoagulant effect, acyclovir for the treatment of herpes virus infections, albuterol to prevent bronchospasm, alclometasone to treat itching, redness, crusting, scaling and inflammation, alfentanil for analgesia, allergen extracts for allergic diseases, allopregnanolone for depression, almotriptan for migraine headaches, aloradine and alprazolam for anxiety disorder, alprostadil for erectile dysfunction, amoxicillin to treat bacterial infections, amphetamines for attention-deficit hyperactivity disorder, androstanediol to increase the production of the hormone testosterone, apomorphine for Parkinson's disease, arctigenin for inflammation or control of blood glucose levels, articaine for anesthesia, ascorbic acid as a nutraceutical, asenapine for bipolar disorder, atomoxetine for the treatment of attention deficit hyperactivity disorder, atorvastatin for the treatment of high cholesterol, atropine for management of secretions in the respiratory tract or gastrointestinal tract, azathioprine for autoimmune diseases, azelastine for respiratory allergies, barbiturate treating seizure disorder, beclomethasone for asthma and chronic obstructive pulmonary disease, bisphosphonates for osteopenia or osteoporosis, budesonide for asthma and inflammation in the respiratory tract, bupivacaine as local anesthetic, buprenorphine as a painkiller agent, bupropion for depression, caffeine for mental alertness, headache, migraine, athletic performance, memory, and obesity, cannabidiol for the treatment of seizures, captopril for hypertension, chlordiazepoxide for anxiety and alcohol withdrawal, chloropheniramine for allergy, chlorpromazine to treat anxiety, mania, psychosis and schizophrenia, cholecalciferol as dietary supplement, clonidine for hypertension, clorazepate for skeletal muscle relaxant, clorazepic acid to treat anxiety, partial seizures, and alcohol withdrawal, cobalamin as dietary supplement, cortisone for asthma and allergies, cromolyn for allergic rhinitis, cyclobenzaprine for painful musculoskeletal conditions, desmopressin for treatment of diabetes insipidus, dexamethasone for severe allergies and asthma, dextroamphetamine to treat attention-deficit hyperactivity disorder and sleep disorder, diazepam anxiety disorder, dihydroergotamine for migraine headaches, dinoprostone for the evacuation of uterine contents and labor induction, donepezil for treatment of dementia, econazole as antifungal agent, eletriptan for migraine headaches, endorphins analgesic and for management of opioid withdrawal, enkephalins as pain killer agent, ephedrine for hypotension, epinephrine for anaphylaxis, ergoloid for cognitive disorders, ergonovine for haemorrhage, ergotamine for migraine, estazolam for sleep disorder, estradiol for menopausal symptoms, estropipate for menopause related issues, etonogestrel for birth control, fentanyl for pain condition, fluconazole as an antifungal agents for urinogenital tract infections, fludrocortisone for adrenocortical insufficiency, fluoxetine for depression, flurazepam for sleep disorder, fluticasone for asthma, frovatriptan for migraine, furosemide to treat conditions with volume overload and edema, gabapentin for neuropathic pain, ganaxolone to treat status epilepticus, gentamicin for bacterial infection treatment, glimepiride for treatment of type 2 diabetes mellitus, glucagon for hypoglycemia, guaifenesin for respiratory congestion, halazepam for anxiety disorders, heparin for anticoagulant therapy, hydrocortisone for inflammation, hyoscyamine for decreasing the motion of the stomach and intestines, ibuprofen anti-inflammatory, antipyretic and pain killer agent, insulin for diabetes type 1 and 2 treatment, ipratropium for chronic obstructive pulmonary disease, isoproterenol to treat bradycardia conditions, ivermectin as antiparasitic agent, ketamine as anesthetic agent, ketoprofen as analgesic agent, labetalol for hypertension, levothyroxine for hypothyroidism, lidocaine for local anesthesia, lofentanil for chronic pain, loratadine for allergies, lorazepam for anxiety disorders, magnesium for the management of preterm labor, melatonin for sleep disorder, mepivacaine for local anesthesia, metformin treatment of type 2 diabetes and gestational diabetes, methadone for pain management, methylphenidate for attention deficit hyperactivity disorder, methysergide for migraines, methyltestosterone for testosterone deficiency, metoclopramide to treat nausea and vomiting, metoprolol to treat angina, metronidazole to treat bacterial and parasitic infections, midazolam for the treatment of status epilepticus, misoprostol for gastric ulcers, mometasone for the treatment of nasal polyps, morphine for pain management, nadolol for angina pectoris, nalbuphine for respiratory depression, naltrexone to treat alcohol use disorder and opioid dependence, naproxen for pain in joints and muscles, naratriptan for migraine, neuropeptide Y for the treatment of post-traumatic stress disorder, nicotine for nicotine replacement therapies, nifedipine for the management of vasospastic angina, nimesulide for pain and fever management, nitroglycerin for angina pectoris, norethindrone for amenorrhea management, novocaine for local anesthesia, nystatin as an antifungal agent, olanzapine for schizophrenia and bipolar disorder treatment, ondansetron to prevent nausea and vomiting, opiates for the treatment of opioid dependence, oxycodone for the management of severe pain, oxymetazoline to treat nasal congestion and rosacea, oxytocic for strengthening uterine contractions, paracetamol for pain and fever management, pentobarbital for convulsive episodes, phenylephrine for nasal congestion, phytonadione for prophylaxis and treatment of vitamin K-deficiency bleeding, prazosin to treat hypertension, prednisone for anti-inflammatory effects, pregabalin to treat neuropathic pain, pregnenolone for hormone replacement, prilocaine for local anesthesia, promethazine allergic rhinitis, propofol for sedation, propranolol for the treatment of essential tremors and migraine, prostaglandin for the evacuation of uterine contents and labor induction, pyridoxine to treat nausea and vomiting of pregnancy, salmeterol for management and treatment of asthma and chronic obstructive pulmonary disease, scopolamine for postoperative nausea and vomiting, segesterone as hormonal contraceptive, selegiline for Parkinson disease and major depressive disorder treatment, sildenafil erectile dysfunction, sufentanil as analgesic adjunct, sumatriptan for acute treatment of migraine, temazepam for the short-term treatment of insomnia, terbutaline for bronchospasm, tetracaine as local anesthetic agent, thiamine for the treatment of thiamine deficiency, timolol to treat hypertension and glaucoma, tinidazole for the treatment of bacterial vaginosis, tocopherol for the treatment of tocopherol deficiency, tramadol for the management of pain, triazolam for sleep disorder, tryptophan for sleeping difficulties, premenstrual syndrome, stress, depression and alcohol and drug abuse, vardenafil to treat erectile dysfunction, varenicline for smoking cessation treatment, verapamil for angina, zolmitriptan for migraine headaches, zolpidem for sleep disorder.

All such pharmaceutical ingredients are known in the field and the skilled person is able to choose the suitable ingredient in view of the desired application of the device of the invention. The skilled person is also able to choose the suitable dosage of the active ingredient in view of the conditions of the subject and of the therapeutical application.

Depending on the desired medical application the device of the invention can be applied in contact with a specific kind of mucosa. In a preferred embodiment, the device is suitable for the delivery of drug to oral, nasal or urogenital mucosa. In this embodiment, the device is placed in contact with said oral, nasal or urogenital mucosa.

The activity of the device can be local, for example if applied on urogenital mucosae, or systemic, for example if applied to the oral or nasal mucosa, from where the drug can diffuse to reach the blood circulation.

The device is removable and can be applied in contact with the mucosa of a subject through a support adapted for each body region of interest. For example, a device according to the invention for application to the oral mucosa can be clopped to a removable orthodontic appliance. Such removable orthodontic appliance can be commercially obtained or can be manufactured according to methods commonly known in the art (Del Rossi and Leyte-Vidal, 2007). For example, it can be manufactured specifically for mucosa drug delivery from thermoplastic polymers, which can be soften when immersed in hot water and then molded to a new shape to adapt to the user dental arch thus obtaining a customized removable orthodontic appliance allowing an appropriately positioning of the device to either buccal or sub-lingual mucosa.

In an embodiment, a device according to the invention for application to the nasal mucosa can be clopped to a respiratory wearable device which can be commercially obtained or manufactured according to methods commonly known in the art (Massaroni et al., 2019). Respiratory wearable device may comprise a catheter tip wherein the drug delivery device of the invention can be placed. The respiratory wearable device can be placed in the right or left nostril and the catheter tip reaches the nasal mucosa.

A device according to the invention for application to the urogenital mucosa can be clopped to removable urogenital apparatus, which can be commercially obtained or manufactured according to methods commonly known in the art. For example, a device according to the invention for application to the male urethral mucosa can be clopped to the tip of an intra-urethral applicator (Padma-Nathan et al., 1997), which can be placed into the urethra allowing the device to be in contact to the urethral mucosa. A device according to the invention for vaginal application can be clopped to a elastomeric polymers vaginal ring (Morrow et a., 2011) and placed in the vaginal canal for vaginal mucosa drug delivery.

The invention will be now described by the following examples.

### EXAMPLES

### Example 1

### Methodology

### Materials

Caffeine (CAF), graphene oxide (GO), GO conjugated with CAF coated with chitosan (CH), ibuprofen (IBU) and ferric sulfate (Fe3SO4) reduced graphene oxide (rGO) conjugated with CAF provided by Federal University of Sao Paulo - Unifesp - Brazil, along with sodium chloride and dimethyl sulfoxide were used in the present study. The concentration of drugs, CAF and IBU were at 2mg/mL and GO or rGO at 0.5mg/mL. Arduino Uno microcontroller, membrane filter with a pore size of 0.45 µm and polypropylene reservoir were also used to fabricate the device.

### Preparation of electrolyte solutions

Sodium chloride (NaCl) was prepared at 0.9% in distilled water. Dimethyl sulfoxide (DMSO) at 0.25M was prepared in sodium chloride 0.9% solution.

### Device assembly

To fabricate the drug delivery device according to the invention a commercialized reservoir was used as a solution container and the place where the electrolysis were performed. An Arduino microcontroller board was used to perform the electrochemical process as described by Crespo et al., 2021. For that, a portable Arduino Uno microcontroller, connected through Wi-Fi to a notebook computer, with boards operating with digital outputs, which go up to 5V, capable of producing a programmable output voltage with the help of a voltage shifter circuit, was configurated using a custom software, written in Visual C++. Graphite counter electrode and working electrode were placed in the opposite walls of device reservoir. All electrodes were in contact with the GO dispersion, that was placed inside the reservoir, along with either NaCl or DMSO electrolyte solution. Finally, a membrane filter with a pore size of 0.45 µm was placed in the bottom of reservoir.

### Experimental procedure

Experiments were performed inside the reservoir containing graphite as counter and work electrodes connected to a portable Arduino Uno microcontroller. All electrodes were immersed in dispersed graphene conjugated with active ingredients and electrolyte. Therefore, NaCl and/or DMSO solutions were used as electrolyte. Based on previously experiments, electrochemical performances were evaluated by applying chronoamperometry under a low given potential of 1.2 V during different periods.

The electrochemical reactions under the given potential was performed, to measure the redox potential. The current variation was measured along the time until 5 minutes in presence of water dispersed graphene oxide loaded with either IBU (GO-IBU), or CAF (GO-CAF), in the last case coated with CH (GO-CH-CAF); and reduced graphene oxide, functionalized with Fe₃SO₄, conjugated with caffeine (rGO-F-CAF).

To induce an electrolytic water splitting, known to produce hydrogen at the cathode electrode and oxygen in the anode one, a potential was applied during 5 minutes (Wang et al., 2021). Then, the capability of graphene oxide to release active ingredients under water electrolysis were evaluated. After that, solution containing caffeine and electrolyte was filtered and analysed by Fourier Transform Infrared Spectroscopy (FTIR) to identify the presence of caffeine in the sample. The analyses were performed in a FTIR Spectrum One (Perkin Elmer), using the liquid film method, in the range of 4,000-550 cm-1, with resolution of 4 cm-1 and 32 scans.

All experimental procedure were performed at room temperature.

### Results and Discussion

### Controlled-Release of Drug

We designed, fabricated, and tested a drug delivery device with graphene oxide (GO) and reduced graphene oxide (rGO) nanosheets as nanocarrier for drug delivery applications upon electrochemical reaction. Indeed, graphene-based nanomaterials are being investigated for therapeutic applications to support the delivery of active pharmaceutical ingredients more efficiently (Cellot et al., 2022). Graphene derivatives such as GO and rGO consist of carbon, oxygen, and hydrogen atoms suitable for non-covalent binding of drugs. Thus, drugs can be linked to their surface by physical adsorption through electrostatic interactions, van der Waals forces or π-π stacking interactions (Georgakilas et al., 2016).

In the present study, GO were conjugated with different active ingredients, such as CAF and IBU, additionally GO-CAF was coated with CH by non-covalent bonds. Moreover, rGO were covalently functionalized with ferric sulfate and conjugated with CAF by non-covalent bonds.

Electrically controlled release of drugs was investigated by applying an electrical potential into the reservoir for different periods of time (5 minutes) in presence of either NaCl physiological solution or NaCl/DMSO vehicle used as a supporting electrolyte solution. Electrolytes play a key role in electrolysis because they increase the conductivity of the water, which allows a greater flow of ions through the solution. Thus, the presence of saline solution leads to a more efficient and effective electrolysis process. Regarding to DMSO, a polar aprotic solvent that can dissolve both water-soluble and water-insoluble substances (Kazi and Dehghan, 2020), it was used as a surfactant excipient, which can increase hydrophilicity of caffeine. In some electrolysis experiments, DMSO is used also to control the reaction kinetics by either donating or accepting electrons (Butler, 1967).

An electric current (+1.2 V) was applied into the reservoir containing GO-CAF, GO-CH-CAF, rGO-F-CAF or GO-IBU water dispersion and the electrolyte solution, inducing water electrolysis. In such condition, water molecules can split into hydrogen and oxygen, with hydrogen formed at the cathode and oxygen at the anode (Wang et al., 2021). The hydrogen gas formed may break the pi bonds through the hydrogenation reactions (Weber et al., 2019), enabling the hydrogen atoms to be adsorbed to GO surface and after breaking the pi interaction with drugs. Besides, hydrogen-rich water has the capability of reducing the amount of oxygen molecule from GO, resulting in an enhancement of its electrical conductivity property (Akhavan et al., 2015). Whereas drugs are released into the solution by desorption process. In a matter of fact, electrochemical reaction can determine the release of adsorbed drug molecules from the graphene-based nanomaterial through desorption (Weaver et al., 2014; He et al., 2017; Teodorescu et al., 2015).

In the present work, chronoamperometry was employed for the evaluation of drug release from GO nanosheets over time. By measuring the current over time, it is possible to obtain information about the redox reaction and the stability of the reaction products (Nishiumi et al., 2005).

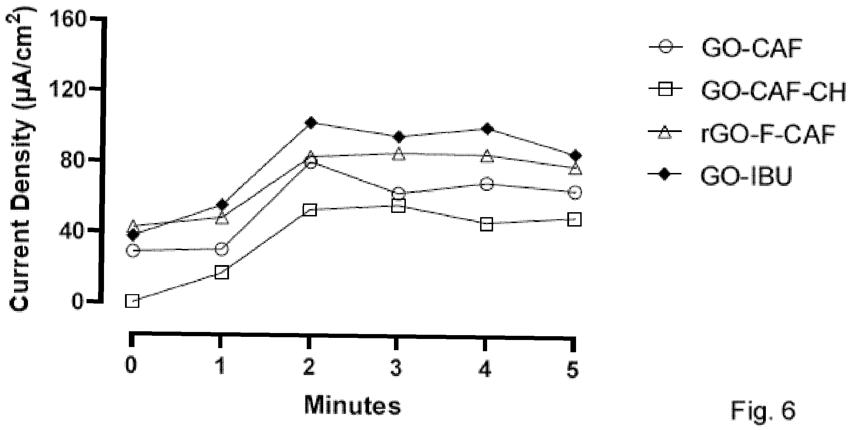

Results showed that the current density increases over the time upon electrochemical reaction characterizing the effective diffusion of drugs, compared to the first measure (time 0; according to the paired T test; t(263)= 2.54; p < 0.05; Figure 6). The CAF release from GO upon electrochemical reaction resulted in a booster of the current, since the first minute, and reached a plateau up to 2 minutes. When GO-CAF was coated with CH, the CAF release also started in the first minute (p<0.05). However the peak current density was lower compared to the GO-CAF sample (p<0.05, according to the unpaired T test), suggesting a slow release. On the other hand, CAF released from rGO-F complex resulted in an enhancement of density current compared to GO-CAF sample (p<0.05, according to the unpaired T test). Apart from CAF, the present delivery system was also tested for IBU release. Even in this case, electrochemical reaction resulted in an increase of the current density over the time, since the first minute (p<0.05, according to the paired T test). Altogether, our results demonstrate a modulatory electro-responsive release of cargo from GO nanosheets.

Our results are in agreement with the electrochemically controlled delivery approach, where graphene platform pre-coated with aniline groups by covalent bonds was shown to control the cargo release upon a low voltage (Hou et al., 2020) that is an advantage for clinical application (Fish and Geddes, 2009).

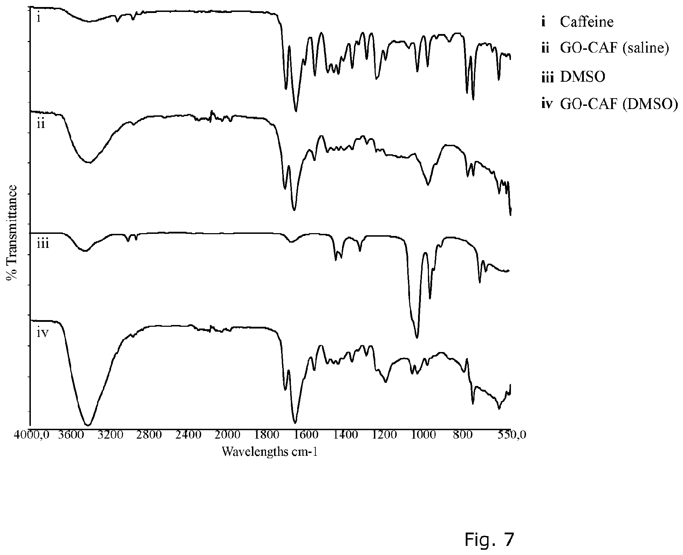

To evaluate the impact of a long-term electrolysis condition, solutions containing CAF released in response to the applied potential, during 5 minutes, were filtered and posteriorly analyzed by IFRT (Figure 7).

Membrane filtration is a process of separating particles in liquid solutions used in a wide range of applications. By applying external pressure, smaller molecules can pass through the membrane filter, retaining larger particles (Cevallos-Mendoza et al., 2022). In the present study, the pressure was applied by the gas formed inside the reservoir. The electrolysis of water is a transformation process of liquid to gas (Xu et al., 2019), useful to convert electrical energy to pressure-volume changes (Cameron and Freund, 2002). Such process may generates sufficient pressure within the drug reservoir, accelerating the transport of caffeine through the membrane filter. In that regard, filtering process was aimed to permit the passage of caffeine and the electrolyte solution, but not graphene oxide nanosheets, that are retained due to their big lateral size work (Tang et al., 2016). By blocking the passage of graphene oxide it's possible to prevent potential toxicity. Despite the fact that graphene oxide shows good biocompatibility and no cytotoxicity (Rauti et al., 2016; Biagioni et al., 2021), the systemic distribution can cause organ toxicity by accumulation mechanism (Zhang et al., 2011). Therefore, filtration process prevents the toxicity effect caused by graphene-based material.

Regarding the analysis, FTIR-spectra showed characteristic peaks of caffeine in the spectral range of 612 - 1701 cm-1 after 5 minutes of applied voltage in presence of either saline of DMSO (Figure 7). Cyclic voltammetry curves of caffeine showed that caffeine undergoes to oxidation process during electrochemical process. In such condition, caffeine oxidation peak potential is around +1.3 V / +1.4 V, and an irreversible oxidation peak potential is over 1.4 V, depending on the electrochemical system used (Hernandez-Aldave et al., 2019; Svorc, 2013). To avoid the irreversible oxidation process, the experiments were performed with the potential of the working electrode at 1.2 V.

Thus, we demonstrated that the capability of graphene oxide to delivery its cargo (caffeine) can be determined by electrical stimulus upon a low voltage and short period of time. The present strategy is not limited to caffeine. Instead, it may be applied for organic and inorganic molecules, polar, apolar aromatic or non-aromatic pharmaceutical ingredients as well. Because graphene can be functionalized by binding different chemical groups to its surface, as demonstrated in previous works (Mancilla et al., 2020; Wang et a., 2013; Kavitha et al., 2013; Liu et al., 2013; Park et a., 2014; Servant et al., 2014).

### Conclusion

Summarizing, we have fabricated a remote-controlled drug delivery device that enable the releasing of active pharmaceutical ingredients in response to an applied electric potential. By using graphene oxide as a platform for loading agents via non-covalent interaction, it is possible to control the speed and dose of the released agent by electrochemical system. Application of a low voltage breaks the pi-interaction resulting in adsorption of the active pharmaceutical ingredient, enabling the control of the releasing profile by applying different electric potential in different time before filtering the solution. This system offers an effective application of graphene as drug delivery system, preventing the potential organ toxicity caused by the accumulation of graphene in vital organ when injected systemically.

### References

Abadal, Sergi, Eduard Alarcón, Albert Cabellos-Aparicio, Max Lemme, and Mario Nemirovsky. 2013. "Graphene-Enabled Wireless Communication for Massive Multicore Architectures." IEEE Communications Magazine 51 (11): 137-43. https://doi.org/10.1109/MCOM.2013.6658665.
Abdelhamid, Hani Nasser, and Kamal Hany Hussein. 2021. "Graphene Oxide as a Carrier for Drug Delivery of Methotrexate." Biointerface Research in Applied Chemistry 11 (6): 14726-35. https://doi.org/10.33263/BRIAC116.1472614735.
Ahmad, Md Wasi, Baban Dey, Gautam Sarkhel, Dibyendu Sekhar Bag, and Arup Choudhury. 2019. "Exfoliated Graphene Reinforced Polybenzimidazole Nanocomposite with Improved Electrical, Mechanical and Thermal Properties." Materials Chemistry and Physics 223: 426-33. https://doi.org/10.1016/j.matchemphys.2018.11.026.
AL-Ghananeem Abeer, M. 2009. Compositions And Methods For Transmucosal Delivery Of Lofexidine. WO 2009/120735 A1, issued 2009.
Andrade, Carolina S., Anna Paula S. Godoy, Marcos Antonio Gimenes Benega, Ricardo J.E. Andrade, Rafael Cardoso Andrade, Wellington Marcos Silva, Josué Marciano de Oliveira Cremonezzi, et al. 2021. "Micro Scalable Graphene Oxide Productions Using Controlled Parameters in Bench Reactor." Nanomaterials 11 (8): 1-11. https://doi.org/10.3390/nano11081975.
Arthi G, Paulchamy B, and Lignesh BD. 2015. "A Simple Approach to Stepwise Synthesis of Graphene Oxide Nanomaterial." Journal of Nanomedicine & Nanotechnology 06 (01): 1-4. https://doi.org/10.4172/2157-7439.1000253.
Bardach, Laura, Geduldig James, and Napoli Salvatore. 2007. Intra-oral device for treating obesity. US 20070048347A1, issued 2007.
Betbeder, Didier, Ignacio De Miguel, Roger Kravtzoff, and Major Michel. 2000. Mucosal administration of substances to mammals. US006017513A, issued 2000.
Caldeira, João M.L.P., Joel J.P.C. Rodrigues, João F.R. Garcia, and Isabel de la Torre. 2010. "A New Wireless Biosensor for Intra-Vaginal Temperature Monitoring." Sensors (Switzerland) 10 (11): 10314-27. https://doi.org/10.3390/s101110314.
Castillo-Henriquez, Luis, Mariana Brenes-Acuña, Arianna Castro-Rojas, Rolando Cordero-Salmerón, Mary Lopretti-Correa, and José Roberto Vega-Baudrit. 2020. "Biosensors for the Detection of Bacterial and Viral Clinical Pathogens." Sensors (Switzerland) 20 (23): 1-26. https://doi.org/10.3390/s20236926.
Chen, Xinjian;, Chengke; Luo, Peter; JENSEN, Li; LAN, and Alana; WELM. 2017. A nanomaterial complex comprising graphene oxide associated with a therapeutic agent and methods of use. WO 2017/066583 A1, issued 2017.
Davis, Stanley S., and Lisbeth Illum. 2003. "Absorption Enhancers for Nasal Drug Delivery." Clinical Pharmacokinetics 42 (13): 1107-28. https://doi.org/10.2165/00003088-200342130-00003.
Fadeel, Bengt, Cyrill Bussy, Sonia Merino, Ester Vázquez, Emmanuel Flahaut, Florence Mouchet, Lauris Evariste, et al. 2018. "Safety Assessment of Graphene-Based Materials: Focus on Human Health and the Environment." ACS Nano 12 (11): 10582-620. https://doi.org/10.1021/acsnano.8b04758.
Farra R, Sheppard NF Jr, McCabe L, Neer RM, Anderson JM, Santini JT Jr, Cima MJ, Langer R. First-in-human testing of a wirelessly controlled drug delivery microchip. Sci Transl Med. 2012 Feb 22;4(122):122ra21. doi: 10.1126/scitranslmed.3003276. Epub 2012 Feb 16. PMID: 22344516.
Fasciani, Chiara, Anabel E. Lanterna, Javier B. Giorgi, and Juan C. Scaiano. 2017. "Visible Light Production of Hydrogen by Ablated Graphene: Water Splitting or Carbon Gasification?" Joumal of the American Chemical Society 139 (32): 11024-27. https://doi.org/10.1021/jacs.7b06570.
Fei, Yuhuan, Siyuan Fang, and Yun Hang Hu. 2020. "Synthesis, Properties and Potential Applications of Hydrogenated Graphene." Chemical Engineering Journal 397 (May): 125408. https://doi.org/10.1016/j.cej.2020.125408.
Furniturewalla, Abbas, Matthew Chan, Jianye Sui, Karan Ahuja, and Mehdi Javanmard. 2018. "Fully Integrated Wearable Impedance Cytometry Platform on Flexible Circuit Board with Online Smartphone Readout." Microsystems and Nanoengineering 4 (1). https://doi.org/10.1038/s41378-018-0019-0.
Georgakilas, Vasilios, Jitendra N. Tiwari, K. Christian Kemp, Jason A. Perman, Athanasios B. Bourlinos, Kwang S. Kim, and Radek Zboril. 2016. "Noncovalent Functionalization of Graphene and Graphene Oxide for Energy Materials, Biosensing, Catalytic, and Biomedical Applications." Chemical Reviews 116 (9): 5464-5519. https://doi.org/10.1021/acs.chemrev.5b00620.
Guo D., Wu X., Lei J., Xu B., Kometani R., Luo F. 2016. Fabrication of Micro/Nanoelectrode Using Focused-ion-Beam Chemical Vapor Deposition, and its Application to Micro-ECDM, Procedia CIRP 42: 733-736.
Hermanson, Christopher, and Amit Rajguru. 2020. System and method for delivering a therapy and sensing a biological activity in the mouth. US010874542B2, issued 2020.
Hou HL , Cardo L , Mancino D , Arnaiz B , Criado A , Prato M . 2020 . Electrochemically controlled cleavage of imine bonds on a graphene platform: towards new electro-responsive hybrids for drug release. Nanoscale. 12(46):23824-23830. doi: 10.1039/d0nr04102e.
Huang, Lijin, Naqing Mao, Qian Yan, Dingwen Zhang, and Qin Shuai. 2020. "Magnetic Covalent Organic Frameworks for the Removal of Diclofenac Sodium from Water." ACS Applied Nano Materials 3 (1): 319-26. https://doi.org/10.1021/acsanm.9b01969.
Jasim, Dhifaf A., Neus Lozano, and Kostas Kostarelos. 2016. "Synthesis of Few-Layered, High-Purity Graphene Oxide Sheets from Different Graphite Sources for Biology." 2D Materials 3 (1): 014006. https://doi.org/10.1088/2053-1583/3/1/014006.
Justin, Richard, and Biqiong Chen. 2018. "Multifunctional Chitosan-Magnetic Graphene Quantum Dot Nanocomposites for the Release of Therapeutics from Detachable and Non-Detachable Biodegradable Microneedle Arrays." Interface Focus 8 (3). https://doi.org/10.1098/rsfs.2017.0055.
Kang, Sung Gu, Min Su Song, Joon Woo Kim, Jung Woo Lee, and Jeonghyun Kim. 2021. "Near-field Communication in Biomedical Applications." Sensors (Switzerland) 21 (3): 1-18. https://doi.org/10.3390/s21030703.
Khalifa, A., Zaeimbashi, M., Zhou, T.X. et al. The development of microfabricated solenoids with magnetic cores for micromagnetic neural stimulation. Microsyst Nanoeng 7, 91 (2021). https://doi.org/10.1038/s41378-021-00320-8
Kim, Jayoung, Somayeh Imani, William R. de Araujo, Julian Warchallb, Valdés-Ramirez Gabriela, Paixão Thiago R.L.C., Mercier Patrick P., and Wang Joseph. 2018. "Wearable Salivary Uric Acid Mouthguard Biosensor with Integrated Wireless Electronics." Biosens Bioelectro. 74: 1061-68. https://doi.org/10.1016/j.bios.2015.07.039.
Kim, Junyeon, Dongwook Go, Hanshen Tsai, Daegeun Jo, Kouta Kondou, Hyun Woo Lee, and Yoshichika Otani. 2021. "Nontrivial Torque Generation by Orbital Angular Momentum Injection in Ferromagnetic-Metal/ Cu/Al2 O3 Trilayers." Physical Review B 103 (2): 1-6. https://doi.org/10.1103/PhysRevB.103.1.020407.
Kwon, I.C., Bae, Y.H., Okano, T., Kim, S.W. and Berner, B. (1990), Stimuli sensitive polymers for drug delivery systems. Makromolekulare Chemie. Macromolecular Symposia, 33: 265-277. https://doi.org/10.1002/masy.19900330123
Liu, Jilei, Huanping Yang, Saw Giek Zhen, Chee Kok Poh, Alok Chaurasia, Jingshan Luo, Xiangyang Wu, et al. 2013. "A Green Approach to the Synthesis of High-Quality Graphene Oxide Flakes via Electrochemical Exfoliation of Pencil Core." RSC Advances 3 (29): 11745-50. https://doi.org/10.1039/c3ra41366g.
Lukas, Christopher J, Farah B Yahya, Jacob Breiholz, Abhishek Roy, Xing Chen, Harsh N Patel, Senior Member, et al. 2019. "Post-Processing SiP for Wearable Applications." IEEE Transactions on Biomedical Circuits and Systems 13 (2): 271-81.
Martorelli, Massimo, Salvatore Gerbino, Michele Giudice, and Pietro Ausiello. 2013. "A Comparison between Customized Clear and Removable Orthodontic Appliances Manufactured Using RP and CNC Techniques." Dental Materials 29 (2): e1-10. https://doi.org/10.1016/j.dental.2012.10.011.
Masoudipour, Elham, Soheila Kashanian, and Nasim Maleki. 2017. "A Targeted Drug Delivery System Based on Dopamine Functionalized Nano Graphene Oxide." Chemical Physics Letters 668: 56-63. https://doi.org/10.1016/j.cplett.2016.12.019.
Massaroni, Carlo, Andrea Nicolo, Michele Girardi, Angelica La Camera, Emiliano Schena, Massimo Sacchetti, Sergio Silvestri, and Fabrizio Taffoni. 2019. "Validation of a Wearable Device and an Algorithm for Respiratory Monitoring during Exercise." IEEE Sensors Journal 19 (12): 4652-59. https://doi.org/10.1109/JSEN.2019.2899658.
Morrow, Ryan J, A David Woolfson, Louise Donnelly, and Rhonda Curran. 2013. "Europe PMC Funders Group Sustained Release of Proteins from a Modified Vaginal Ring Device" 77 (1): 3-10. https://doi.org/10.1016/j.ejpb.2010.10.010.Sustained.
Ou, Lingling, Bin Song, Huimin Liang, Jia Liu, Xiaoli Feng, Bin Deng, Ting Sun, and Longquan Shao. 2016. "Toxicity of Graphene-Family Nanoparticles: A General Review of the Origins and Mechanisms." Particle and Fibre Toxicology 13 (1). https://doi.org/10.1186/s12989-016-0168-y.
Padma-Nathan, Harin, Hellstrom Wayne J.G., Kaiser Fran E., Labasky Richard F., Lue Tom F., Nolten Wolfram E., Norwood Paul C., et al. 1997. "Treatment Of Men With Erectile Dysfunction With Transurethral Alprostadil." The New England Journal of Medicine 336 (1): 1-7.
Paredes, J. I., S. Villar-Rodil, A. Martinez-Alonso, and J. M.D. Tascón. 2008. "Graphene Oxide Dispersions in Organic Solvents." Langmuir 24 (19): 10560-64. https://doi.org/10.1021/1a801744a.
Passador, F. R., A. Ruvolo-Filho, and L. A. Pessan. 2017. Nanocomposites of Polymer Matrices and Lamellar Clays. Nanostructures. Elsevier Inc. https://doi.org/10.1016/B978-0-323-49782-4.00007-3.
Pei, Xibo, Zhou Zhu, Zhoujie Gan, Junyu Chen, Xin Zhang, Xinting Cheng, Qianbing Wan, and Jian Wang. 2020. "PEGylated Nano-Graphene Oxide as a Nanocarrier for Delivering Mixed Anticancer Drugs to Improve Anticancer Activity." Scientific Reports 10 (1): 1-15. https://doi.org/10.1038/s41598-020-59624-w.
Perlman, Harel, Tsion Eisenfeld, and Avi Karsenty. 2021. "Performance Enhancement and Applications Review of Nano Light Emitting Device (Led)." Nanomaterials 11 (1): 1-27. https://doi.org/10.3390/nano11010023.
Puthalath, Koroth Raghuprasad. 2014. Oral transmucosal drug delivery device. US 2014/0207111A1, issued 2014.
Reddy, Sathish, Li Song, Yuyuan Zhao, Rong Zhao, Dongni Wu, Liumin He and Seeram Ramakrishana. 2018 "Reduced graphene oxide-based electrochemically stimulated method for temozolomide delivery." Medical Devices & Sensors 1:e10014.
Reza Radmand. 2019. Removable mandibular pharmaceutical delivery device. US 2019 / 0106812 A1, issued 2019.
Rossi, Gianluca Del, and Marco A. Leyte-Vidal. 2007. "Fabricating a Better Mouthguard. Part I: Factors Influencing Mouthguard Thinning." Dental Traumatology 23 (3): 149-54. https://doi.org/10.1111/j.1600-9657.2006.00436.x.
S. 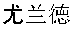 , E. 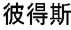 , and S.H.Y. 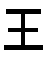 . 2011. Saturating mucosal drug conveyer device and the method for potentiation is permeated including driven by power. CN102641547B 10, issued 2011.
Santini, Inventors John T, Michael J Cima, M A Us, Robert S Langer, Sutherland Asbill, and Brennan Llp. 2006. Microchip drug delivery devices. US 7,070,590 B1, issued 2006.
Santini JR, John T., Michael J. Cima, and Robert S. Langer. 2008. Method for Making Microchip Reservor Device. US 20080047926A1, issued 2008.
Sharma, S., J. Kulkarni, and A. P. Pawar. 2006. "Permeation Enhancers in the Transmucosal Delivery of Macromolecules." Pharmazie 61 (6): 495-504.
Singh, Kuldeep, Anil Ohlan, and S K Dhawan. 2012. "Chapter 3: Polymer-Graphene Nanocomposites : Preparation , Characterization , Properties , and Applications." Intech Open, 37-71.
Slaughter, Gymama, and Tanmay Kulkarni. 2017. "Highly Selective and Sensitive Self-Powered Glucose Sensor Based on Capacitor Circuit." Scientific Reports 7 (1): 1-9. https://doi.org/10.1038/s41598-017-01665-9.
Song, Chang-Woo, and Seung-Yop Lee. 2015. "Design of a Solenoid Actuator with a Magnetic Plunger for Miniaturized Segment Robots" Applied Sciences 5(3): 595-607. https://doi.org/10.3390/app5030595
Stevenson, Hunter, Amanda Bacon, Kathleen Mary Joseph, Wilma Ruth Wanjiku Gwandaru, Ashlesha Bhide, Devangsingh Sankhala, Vikram Narayanan Dhamu, and Shalini Prasad. 2019. "A Rapid Response Electrochemical Biosensor for Detecting Thc In Saliva." Scientific Reports 9 (1): 1-11. https://doi.org/10.1038/s41598-019-49185-y.
Subrahmanyam, K. S., Prashant Kumar, Urmimala Maitra, A. Govindaraj, K. P.S.S. Hembram, Umesh V. Waghmare, and C. N.R. Rao. 2011. "Chemical Storage of Hydrogen in Few-Layer Graphene." Proceedings of the National Academy of Sciences of the United States of America 108 (7): 2674-77. https://doi.org/10.1073/pnas.1019542108.
Uhland, Scott, Eric Peeters, and Serena Wong. 2009. Transmucosal drug delivery device including electroporation. EP2311522B1, issued 2009.
Vasisht, Niraj, and Karl D. Kelly. 2021. Pharmaceutical active-containing film delivery device for oral transmucosal administration. US2021030693A1, issued 2021.
Weaver, Cassandra L., Jaclyn M. Larosa, Xiliang Luo, and Xinyan Tracy Cui. 2014. "Electrically Controlled Drug Delivery from Graphene Oxide Nanocomposite Films." ACS Nano 8 (2): 1834-43. https://doi.org/10.1021/nn406223e.
Weichbroth, Pawel. 2020. "Usability of Mobile Applications: A Systematic Literature Study." IEEE Access 8: 55563-77. https://doi.org/10.1109/ACCESS.2020.2981892.
Wolff, Andy, and Ben Beiski. 2004. Oral Devices and Methods For Controlled Drug Delivery. Wo 2004/069076 a2, issued 2004.
Wu, L., Wang, J., Gao, N. et al. 2015. Electrically pulsatile responsive drug delivery platform for treatment of Alzheimer's disease. Nano Res. 8:2400-2414. https://doi.org/10.1007/s12274-015-0750-x
Yang, Song, Tian Zeng, Yaling Li, Jun Liu, Qian Chen, Ji Zhou, Yong Ye, and Bin Tang. 2015. "Preparation of Graphene Oxide Decorated Fe3O4@SiO2 Nanocomposites with Superior Adsorption Capacity and SERS Detection for Organic Dyes." Joumal of Nanomaterials 2015. https://doi.org/10.1155/2015/817924.
Yates, Alayne. 2000. Gum pad for delivery of medication to mucosal tissues. WO 00/62764 A1, issued 2000.
Yi, Jongdarm, Goeun Choe, Junggeon Park, and Jae Young Lee. 2020. "Graphene Oxide-Incorporated Hydrogels for Biomedical Applications." Polymer Journal 52 (8): 823-37. https://doi.org/10.1038/s41428-020-0350-9.
CN109464397A, issued 2019. A kind of graphene oxide multiple medicine based on biomineralization is total to loading system and its preparation method and application.
Butler, James N. Electrochemistry in dimethyl sulfoxide, Journal of Electroanalytical Chemistry and Interfacial Electrochemistry, 1967. 14(1): 89-116..
Cameron CG, Freund MS. Electrolytic actuators: alternative, high-performance, material-based devices. Proc Natl Acad Sci U S A. 2002 Jun 11;99(12):7827-31. doi: 10.1073/pnas.122212299.
Cellot, G., Franceschi Biagioni, A. & Ballerini, L. Nanomedicine and graphene-based materials: advanced technologies for potential treatments of diseases in the developing nervous system. Pediatr Res 92, 71-79 (2022). https://doi.org/10.1038/s41390-021-01681-6
Cevallos-Mendoza J, Amorim CG, Rodriguez-Diaz JM, Montenegro MdCBSM. Removal of Contaminants from Water by Membrane Filtration: A Review. Membranes. 2022; 12(6):570.
Fish RM, Geddes LA. Conduction of electrical current to and through the human body: a review. Eplasty. 2009 Oct 12;9:e44. PMID: 19907637; PMCID: PMC2763825.
Franceschi Biagioni A, Cellot G, Pati E, Lozano N, Ballesteros B, Casani R, Coimbra NC, Kostarelos K, Ballerini L. Graphene oxide prevents lateral amygdala dysfunctional synaptic plasticity and reverts long lasting anxiety behavior in rats. Biomaterials. 2021 Apr;271:120749. doi: 10.1016/j.biomaterials.2021.120749.
Georgakilas, V. et al. Noncovalent functionalization of graphene and graphene oxide for energy materials, biosensing, catalytic, and biomedical applications. Chem. Rev. 116, 5464-5519 (2016).
He, Lijie, Sarkar, Sujoy, Barras, Alexandre, Boukherroub, Rabah, Szunerits, Sabine, Mandler, Daniel. 2017. Electrochemically stimulated drug release from flexible electrodes coated electrophoretically with doxorubicin loaded reduced graphene oxide. Chem. Commun., 2017,53, 4022-4025.
Hemandez-Aldave S, Tarat A, McGettrick JD, Bertoncello P. Voltammetric Detection of Caffeine in Beverages at Nafion/Graphite Nanoplatelets Layer-by-Layer Films. Nanomaterials (Basel). 2019 Feb 7;9(2):221. doi: 10.3390/nano9020221. PMID: 30736450; PMCID: PMC6410159.
Hou HL , Cardo L , Mancino D , Arnaiz B , Criado A , Prato M . Electrochemically controlled cleavage of imine bonds on a graphene platform: towards new electro-responsive hybrids for drug release. Nanoscale. 2020 Dec 8;12(46):23824-23830. doi: 10.1039/d0nr04102e. PMID: 33237058.
Kavitha, T., Haider Abdi, S. I. & Park, S. Y. PH-Sensitive nanocargo based on smart polymer functionalized graphene oxide for site-specific drug delivery. Phys. Chem. Chem. Phys. 15, 5176-5185 (2013).
Kavitha, T., Haider Abdi, S. I. & Park, S. Y. PH-Sensitive nanocargo based on smart polymer functionalized graphene oxide for site-specific drug delivery. Phys. Chem. Chem. Phys. 15, 5176-5185 (2013).
Kazi MS, Dehghan MH. Mapping the Impact of a Polar Aprotic Solvent on the Microstructure and Dynamic Phase Transition in Glycerol Monooleate/Oleic Acid Systems. Turk J Pharm Sci. 2020 Jun;17(3):307-318. doi: 10.4274/tjps.galenos.2019.26096.
Liu, G. et al. Transferrin modified graphene oxide for glioma-targeted drug delivery: in vitro and in vivo evaluations. ACS Appl. Mater. Interfaces (2013) 5, 6909-6914.
Mancilla, T. R., Iskra, B. & Aune, G. J. Doxorubicin-induced cardiomyopathy in children. Compr. Physiol. 9, 905-931 (2020).
Nishiumi, Toyohiko, Abdul, M, Aoki, K. Determination of the number of electrons by chronoamperometry at small electrodes. Electrochemistry Communications 7 (2005) 1213-1217.
O. Akhavan, R. Azimirad, H.T. Gholizadeh, F. Ghorbani, Hydrogen-rich water for green reduction of graphene oxide suspensions, Intemational Journal of Hydrogen Energy, 2015. 40 (16): 5553-5560.
Ok-Kyung Park, Seon-Guk Kim, Nam-Ho You, Bon-Cheol Ku, David Hui, Joong Hee Lee. Synthesis and properties of iodo functionalized graphene oxide/polyimide nanocomposites, Composites Part B: Engineering 2014. 56: 365-371.
Park, Ok-Kyung; Kim, Seon-Guk; You, Nam-Ho; Ku, Bon-Cheol; Hui, David; Lee, Joong Hee. Synthesis and properties of iodo functionalized graphene oxide/polyimide nanocomposites, Composites Part B: Engineering. 2014, 56, 365-371. Doi:
Periyasamy S, Muthuchamy M. Electrochemical degradation of psychoactive drug caffeine in aqueous solution using graphite electrode. Environ Technol. 2018 Sep;39(18):2373-2381. doi: 10.1080/09593330.2017.1355932. Epub 2017 Oct 4. PMID: 28705089.
Rauti R, Lozano N, León V, Scaini D, Musto M, Rago I, Ulloa Severino FP, Fabbro A, Casalis L, Vázquez E, Kostarelos K, Prato M, Ballerini L. Graphene Oxide Nanosheets Reshape Synaptic Function in Cultured Brain Networks. ACS Nano. 2016 Apr 26;10(4):4459-71. doi: 10.1021/acsnano.6b00130. Epub 2016 Apr 6. PMID: 27030936.
Servant A, Leon V, Jasim D, Methven L, Limousin P, Fernandez-Pacheco EV, Prato M, Kostarelos K. Graphene-based electroresponsive scaffolds as polymeric implants for on-demand drug delivery. Adv Healthc Mater. 2014. 3(8):1334-43. doi: 10.1002/adhm.201400016.
Švorc, L'. Determination of Caffeine: A Comprehensive Review on Electrochemical Methods. Chemistry 2013. Int. J. Electrochem. Sci., 8 (2013) 5755 - 5773
Tang B, Zhang L, Li R, Wu J, Hedhili MN, Wang P. Are vacuum-filtrated reduced graphene oxide membranes symmetric? Nanoscale. 2016 Jan 14;8(2):1108-16. doi: 10.1039/c5nr06797a. PMID: 26667828.
Teodorescu F, Rolland L, Ramarao V, Abderrahmani A, Mandler D, Boukherroub R, Szunerits S. Electrochemically triggered release of human insulin from an insulin-impregnated reduced graphene oxide modified electrode. Chem Commun (Camb). 2015 Sep 28;51(75):14167-70. doi: 10.1039/c5cc05539c. Epub 2015 Aug 10. PMID: 26257079.
Wang, S., Lu, A. & Zhong, CJ. Hydrogen production from water electrolysis: role of catalysts. Nano Convergence 8, 4 (2021).
Wang, Y. et al. Multifunctional mesoporous silica-coated graphene nanosheet used for chemo-photothermal synergistic targeted therapy of glioma. J. Am. Chem. Soc. 135, 4799-4804 (2013).
Weaver CL, LaRosa JM, Luo X, Cui XT. Electrically controlled drug delivery from graphene oxide nanocomposite films. ACS Nano. 2014 Feb 25;8(2):1834-43. doi: 10.1021/nn406223e. Epub 2014 Jan 17. PMID: 24428340; PMCID: PMC4004293.
Xiong, S. et al. Targeted graphene oxide for drug delivery as a therapeutic nanoplatform against Parkinson's disease. Biomater. Sci. 9, 1705-1715 (2021).
Xu. You, Xu. You, Zhang. Bin. (2019). Recent Advances in Electrochemical Hydrogen Production from Water Assisted by Altemative Oxidation Reactions. 6(13):3214-3226. doi: 10.1002/CELC.201900675
Zhang, Xiaoyong, Yin, Jilei Peng, Cheng, Hu, Weiqing, Zhu, Zhiyon, Li, Wenxin, Fan, Chunhai, Huang, Qing. Distribution and biocompatibility studies of graphene oxide in mice after intravenous administration, Carbon, 2011. 49(3): 986-995.

## Claims

1. A drug delivery device for mucosal and/or transmucosal drug delivery comprising at least one drug reservoir, said drug reservoir comprising:
- at least one active pharmaceutical ingredient pre-loaded on graphene and/or its derivatives, such as graphene oxide;
- a microcontroller able to deliver electrical current to the inner portion of said drug reservoir and able to connect to an electronic device,
wherein the release of said active pharmaceutical ingredient from said drug reservoir is controlled by said microcontroller, and wherein:
said drug reservoir comprises a first filter on the side of the drug reservoir opposite to the microcontroller, said filter being provided with nanopores allowing the passage of the active pharmaceutical ingredient but not the passage of graphene and/or its derivatives, or
graphene and/or its derivatives are functionalised with one or more magnetic nanoparticles forming graphene-based magnetic nanoparticles and the microcontroller is able to deliver a current-controlled nanomagnetic field which prevents the graphene-based magnetic nanoparticles to reach the mucosa
wherein graphene derivatives are selected from the group consisting of graphene oxide, reduced graphene oxide, hydrogenated graphene, graphane, graphone, graphyne, graphdiyne,
fluorographene, optionally modified by covalent or non-covalent functionalization with organic or inorganic molecule.

2. The device of claim 1 wherein said pharmaceutical ingredient is pre-loaded on graphene oxide, preferably said graphene oxide being in the form of one or more layers sheets.

3. The device according to claim 1 or 2, wherein said drug reservoir further comprises an electromagnetic element and said microcontroller further delivers a current-controlled magnetic field to the inner portion of said drug reservoir through said electromagnetic element.

4. The device of claim 3 wherein said drug reservoir further comprises a gate placed adjacent to said electromagnetic element and controlled by it, allowing to control the opening and closing of the reservoir.

5. The device of anyone of the preceding claims, wherein said drug reservoir further comprises at least one electrode and said microcontroller delivers electric current to the inner portion of said drug reservoir through said at least one electrode.

6. The device of anyone of the preceding claims wherein the microcontroller is a battery-operated miniaturized digital embedded electronic system.

7. The device of anyone of the preceding claims wherein said drug reservoir has at least one side configured to face the mucosa of a subject and said drug reservoir further comprises a reservoir cap located at the end of the drug reservoir which is configured to face the mucosa.

8. The device of anyone of the preceding claims wherein said device further comprises one or more electrolyte compartment placed adjacent to said drug reservoir and comprising an electrolyte solution.

9. The device of anyone of the preceding claims wherein said drug reservoir further comprises an excipient compartment separated by the compartment comprising the active pharmaceutical ingredient loaded on graphene and/or its derivatives by a second filter, said excipient compartment comprising one or more excipients.

10. The device of anyone of the preceding claims wherein said drug reservoir further comprises a filter cover placed on top of said first filter, said filter cover preferably being flanked by a pair of electrodes which allow to control the time of filtering of the reservoir content.

11. The device of anyone of the preceding claims wherein said microcontroller is or comprises a microprocessor.

12. The device of anyone of the preceding claims wherein the active pharmaceutical ingredient is for the treatment of at least one of the following disorders: gastrointestinal disorders, genitourinary disorders, psychiatric disorder, neurological disorder and nutritional deficiency, including but not limited to adrenal insufficiency, allergy, anxiety disorder, asthma, attention-deficit hyperactivity disorder, bipolar disorder, chronic and acute pain, chronic bronchitis, chronic obstructive pulmonary disease, chronic obstructive pulmonary disease, congestion, cramp, cyclic vomiting syndrome, dementias, dependence syndrome, diabetes mellitus, epilepsy, fever, genitourinary disorder, hypercholesterolemia, hormonal imbalance, hyperglycemia, hypersalivation, hypertension, hypotension, hypothyroidism, infectious disease, inflammatory diseases, menopause, menstrual regulation, migraine, mood disorders, musculoskeletal pain, nausea, neoplasms, obesity, Parkinson's disease, pulmonary disease, respiratory disease, sexual dysfunction, sleep disorder, smoking cessation, vaginal diseases, vomiting.

13. The device of anyone of the preceding claims wherein the active pharmaceutical ingredient is selected from the group consisting of at least one of: acyclovir, albuterol, alclometasone, alfentanil, allergen extracts, allopregnanolone, almotriptan, aloradine, alprazolam, alprostadil, amoxicillin, amphetamines, androstanediol, apomorphine, arctigenin, articaine, ascorbic acid, asenapine, atomoxetine, atorvastatin, atropine, azathioprine, azelastine, barbiturate, beclometasone, bisphosphonates, budesonide, bupivacaine, buprenorphine, bupropion, caffeine, cannabidiol, captopril, carfentanil, chlordiazepoxide, chloropheniramine, chlorpromazine, cholecalciferol, clonidine, clorazepate, clorazepic acid, cobalamin, cortisone, cromolyn, cyclobenzaprine, desmopressin, dexamethasone, dextroamphetamine, diazepam, diethylcarbamazine, dihydroergotamine, dinoprostone, donepezil, econazole, eletriptan, endorphins, enkephalins, ephedrine, epinephrine, ergoloid, ergonovine, ergotamine, estazolam, estradiol, estropipate, etonogestrel, fentanyl, fluconazole, fludrocortisone, fluoxetine, flurazepam, fluticasone, fluticasone, framycetin, frovatriptan, furosemide, gabapentin, ganaxolone, gentamicin, glimepiride, glucagon, gramicidin, guaifenesin, halazepam, heparin, hydrocortisone, hyoscyamine, ibuprofen, insulin, ipratropium, isoproterenol, ivermectin, ketamine, ketoprofen, labetalol, levothyroxine, lidocaine, lofentanil, loratadine, lorazepam, magnesium, melatonin, mepivacaine, metformin, methadone, methylphenidate, methylsergide, methyltestosterone, metoclopramide, metoprolol, metronidazole, midazolam, midazolam, misoprostol, mometasone, morphine, nadolol, nalbuphine, naloxone, naltrexone, naproxen, naratriptan, nicotine, nifedipine, nimesulide, nitroglycerin, norethindrone, novocaine, olanzapine, ondansetron, opiates, oxycodone, oxycodone, oxymetazoline, oxymetazoline, oxytocic, paracetamol, pentobarbital, phenylephrine, phytonadione, pilocarpine, prazosin, prednisone, pregabalin, pregnanolone, prilocaine, prilocaine, promethazine, propofol, propranolol, prostaglandin, pyridoxine, retinol, rizaptril, salicylic acid, salmeterol, scopolamine, segesterone, selegiline, sildenafil, sufentanil, sumatriptan, temazepam, terbutaline, tetracaine, thiamine, thymosin, timolol, tinidazole, tocopherol, tramadol, triamcinolone, triazolam, tryptophan, vardenafil, varenicline, verapamil, zoledronic acid, zolmitriptan and zolpidem.

14. The device of claim 13 for use in the treatment of at least one of the following disorders or conditions: gastrointestinal disorders, genitourinary disorders, psychiatric disorder, neurological disorder and nutritional deficiency, including but not limited to adrenal insufficiency, allergy, anxiety disorder, asthma, attention-deficit hyperactivity disorder, bipolar disorder, chronic and acute pain, chronic bronchitis, chronic obstructive pulmonary disease, chronic obstructive pulmonary disease, congestion, cramp, cyclic vomiting syndrome, dementias, dependence syndrome, diabetes mellitus, epilepsy, fever, genitourinary disorder, hypercholesterolemia, hormonal imbalance, hyperglycemia, hypersalivation, hypertension, hypotension, hypothyroidism, infectious disease, inflammatory diseases, menopause, menstrual regulation, migraine, mood disorders, musculoskeletal pain, nausea, neoplasms, obesity, Parkinson's disease, pulmonary disease, respiratory disease, sexual dysfunction, sleep disorder, smoking cessation, vaginal diseases, vomiting.

15. The device of anyone of claims 1-13 or the device for use according to claim 14 wherein said device is for drug delivery to oral, nasal or urogenital mucosa, preferably said device further comprising a support adapted for the body region of interest.

## Patentansprüche

1. Vorrichtung zur Verabreichung von Arzneimitteln über die Schleimhaut und/oder transmukosal, umfassend mindestens ein Arzneimittelreservoir, wobei das Arzneimittelreservoir Folgendes umfasst:
- mindestens einen pharmazeutischen Wirkstoff, der auf Graphen und/oder dessen Derivaten, wie etwa Graphenoxid, vorbeladen ist;
- einen Mikrocontroller, der in der Lage ist, elektrischen Strom an den inneren Abschnitt des Arzneimittelreservoirs abzugeben und eine Verbindung zu einer elektronischen Vorrichtung herzustellen,
wobei die Freisetzung des pharmazeutischen Wirkstoffs aus dem Arzneimittelreservoir durch den Mikrocontroller gesteuert wird, und wobei
das Arzneimittelreservoir an der dem Mikrocontroller gegenüberliegenden Seite einen ersten Filter aufweist, wobei dieser Filter mit Nanoporen versehen ist, die den Durchtritt des pharmazeutischen Wirkstoffs, jedoch nicht den Durchtritt von Graphen und/oder dessen Derivaten ermöglichen, oder wobei Graphen und/oder dessen Derivate mit einem oder mehreren magnetischen Nanopartikeln funktionalisiert sind, wodurch magnetische Nanopartikel auf Graphenbasis gebildet werden, und der Mikrocontroller in der Lage ist, ein stromgesteuertes nanomagnetisches Feld abzugeben, das verhindert, dass die magnetischen Nanopartikel auf Graphenbasis die Schleimhaut erreichen,
wobei die Graphen-Derivate aus der Gruppe ausgewählt sind, die aus Graphenoxid, reduziertem Graphenoxid, hydriertem Graphen, Graphan, Graphon, Graphyn, Graphdiyn, Fluorographen besteht, gegebenenfalls modifiziert durch kovalente oder nicht-kovalente Funktionalisierung mit einem organischen oder anorganischen Molekül.

2. Vorrichtung nach Anspruch 1, wobei der pharmazeutische Wirkstoff auf Graphenoxid vorbeladen ist, wobei das Graphenoxid vorzugsweise in Form von ein- oder mehrlagigen Schichten vorliegt.

3. Vorrichtung nach Anspruch 1 oder 2, wobei das Wirkstoffreservoir ferner ein elektromagnetisches Element umfasst und der Mikrocontroller ferner über das elektromagnetische Element ein stromgesteuertes Magnetfeld an den inneren Abschnitt des Wirkstoffreservoirs abgibt.

4. Vorrichtung nach Anspruch 3, wobei das Wirkstoffreservoir ferner eine Sperre umfasst, die benachbart zu dem elektromagnetischen Element angeordnet ist und von diesem gesteuert wird, wodurch das Öffnen und Schließen des Reservoirs gesteuert werden kann.

5. Vorrichtung nach einem der vorhergehenden Ansprüche, wobei das Wirkstoffreservoir ferner mindestens eine Elektrode umfasst und der Mikrocontroller über die mindestens eine Elektrode elektrischen Strom an den inneren Abschnitt des Wirkstoffreservoirs abgibt.

6. Vorrichtung nach einem der vorhergehenden Ansprüche, wobei es sich bei dem Mikrocontroller um ein batteriebetriebenes, miniaturisiertes, digitales eingebettetes elektronisches System handelt.

7. Vorrichtung nach einem der vorhergehenden Ansprüche, wobei das Wirkstoffreservoir mindestens eine Seite aufweist, die so konfiguriert ist, dass sie der Schleimhaut eines Subjekts zugewandt ist, und wobei das Wirkstoffreservoir ferner eine Reservoirkappe umfasst, die an dem Ende des Wirkstoffreservoirs angeordnet ist, das der Schleimhaut zugewandt ist.

8. Vorrichtung nach einem der vorhergehenden Ansprüche, wobei die Vorrichtung ferner eine oder mehrere Elektrolytkammern umfasst, die benachbart zu dem Wirkstoffreservoir angeordnet sind und eine Elektrolytlösung enthalten.

9. Vorrichtung nach einem der vorhergehenden Ansprüche, wobei das Wirkstoffreservoir ferner eine Hilfsstoffkammer umfasst, die durch einen zweiten Filter von der Kammer getrennt ist, welche den auf Graphen und/oder dessen Derivaten geladenen pharmazeutischen Wirkstoff enthält, wobei die Hilfsstoffkammer einen oder mehrere Hilfsstoffe enthält.

10. Vorrichtung nach einem der vorhergehenden Ansprüche, wobei das Wirkstoffreservoir ferner eine Filterabdeckung umfasst, die auf dem ersten Filter angeordnet ist, wobei die Filterabdeckung vorzugsweise von einem Elektrodenpaar flankiert wird, das die Steuerung der Filtrationszeit des Reservoirinhalts ermöglicht.

11. Vorrichtung nach einem der vorhergehenden Ansprüche, wobei der Mikrocontroller ein Mikroprozessor ist oder einen Mikroprozessor umfasst.

12. Vorrichtung nach einem der vorhergehenden Ansprüche, wobei der pharmazeutische Wirkstoff zur Behandlung mindestens einer der folgenden Erkrankungen vorgesehen ist: gastrointestinale Erkrankungen, Erkrankungen des Urogenitalsystems, psychiatrische Erkrankung, neurologische Erkrankung und Nährstoffmangel, einschließlich, jedoch nicht beschränkt auf, Nebenniereninsuffizienz, Allergie, Angststörung, Asthma, Aufmerksamkeitsdefizit- /Hyperaktivitätsstörung, bipolare Störung, chronische und akute Schmerzen, chronische Bronchitis, chronisch obstruktive Lungenerkrankung, chronisch obstruktive Lungenerkrankung, Kongestion, Krämpfe, zyklisches Erbrechenssyndrom, Demenzerkrankungen, Abhängigkeitssyndrom, Diabetes mellitus, Epilepsie, Fieber, Erkrankungen des Urogenitalsystems, Hypercholesterinämie, hormonelles Ungleichgewicht, Hyperglykämie, Hypersalivation, Hypertonie, Hypotonie, Hypothyreose, Infektionskrankheiten, entzündliche Erkrankungen, Menopause, Menstruationsregulierung, Migräne, affektive Störungen, muskuloskelettale Schmerzen, Übelkeit, Neoplasien, Adipositas, Parkinson-Krankheit, Lungenerkrankung, Atemwegserkrankung, sexuelle Dysfunktion, Schlafstörungen, Raucherentwöhnung, vaginale Erkrankungen, Erbrechen.

13. Vorrichtung nach einem der vorhergehenden Ansprüche, wobei der pharmazeutische Wirkstoff ausgewählt ist aus der Gruppe bestehend aus mindestens einem der Folgenden: Aciclovir, Albuterol, Alclometason, Alfentanil, Allergenextrakte, Allopregnanolon, Almotriptan, Aloradin, Alprazolam, Alprostadil, Amoxicillin, Amphetamine, Androstandiol, Apomorphin, Arctigenin, Articain, Ascorbinsäure, Asenapin, Atomoxetin, Atorvastatin, Atropin, Azathioprin, Azelastin, Barbiturate, Beclometason, Bisphosphonate, Budesonid, Bupivacain, Buprenorphin, Bupropion, Koffein, Cannabidiol, Captopril, Carfentanil, Chlordiazepoxid, Chlorpheniramin, Chlorpromazin, Cholecalciferol, Clonidin, Clorazepat, Clorazepinsäure, Cobalamin, Cortison, Cromolyn, Cyclobenzaprin, Desmopressin, Dexamethason, Dextroamphetamin, Diazepam, Diethylcarbamazin, Dihydroergotamin, Dinoproston, Donepezil, Econazol, Eletriptan, Endorphine, Enkephaline, Ephedrin, Epinephrin, Ergoloid, Ergonovin, Ergotamin, Estazolam, Estradiol, Estropipat, Etonogestrel, Fentanyl, Fluconazol, Fludrocortison, Fluoxetin, Flurazepam, Fluticason, Framycetin, Frovatriptan, Furosemid, Gabapentin, Ganaxolon, Gentamicin, Glimepirid, Glucagon, Gramicidin, Guaifenesin, Halazepam, Heparin, Hydrocortison, Hyoscyamin, Ibuprofen, Insulin, Ipratropium, Isoproterenol, Ivermectin, Ketamin, Ketoprofen, Labetalol, Levothyroxin, Lidocain, Lofentanil, Loratadin, Lorazepam, Magnesium, Melatonin, Mepivacain, Metformin, Methadon, Methylphenidat, Methysergid, Methyltestosteron, Metoclopramid, Metoprolol, Metronidazol, Midazolam, Midazolam, Misoprostol, Mometason, Morphin, Nadolol, Nalbuphin, Naloxon, Naltrexon, Naproxen, Naratriptan, Nikotin, Nifedipin, Nimesulid, Nitroglycerin, Norethisteron, Novocain, Olanzapin, Ondansetron, Opiate, Oxycodon, Oxycodon, Oxymetazolin, Oxymetazolin, Oxytocikum, Paracetamol, Pentobarbital, Phenylephrin, Phytonadion, Pilocarpin, Prazosin, Prednison, Pregabalin, Pregnanolon, Prilocain, Prilocain, Promethazin, Propofol, Propranolol, Prostaglandin, Pyridoxin, Retinol, Rizaptril, Salicylsäure, Salmeterol, Scopolamin, Segesteron, Selegilin, Sildenafil, Sufentanil, Sumatriptan, Temazepam, Terbutalin, Tetracain, Thiamin, Thymosin, Timolol, Tinidazol, Tocopherol, Tramadol, Triamcinolon, Triazolam, Tryptophan, Vardenafil, Vareniclin, Verapamil, Zoledronsäure, Zolmitriptan und Zolpidem.

14. Vorrichtung nach Anspruch 13 zur Verwendung bei der Behandlung mindestens einer der folgenden Erkrankungen oder Beschwerden: Magen-Darm-Erkrankungen, Erkrankungen des Urogenitaltrakts, psychiatrische Störung, neurologische Störung und Nährstoffmangel, einschließlich, aber nicht beschränkt auf Nebennierenrindeninsuffizienz, Allergie, Angststörung, Asthma, Aufmerksamkeitsdefizit- /Hyperaktivitätsstörung, bipolare Störung, chronische und akute Schmerzen, chronische Bronchitis, chronisch obstruktive Lungenerkrankung, chronisch obstruktive Lungenerkrankung, Kongestion, Krampf, zyklisches Erbrechens-Syndrom, Demenzen, Abhängigkeitssyndrom, Diabetes mellitus, Epilepsie, Fieber, Erkrankung des Urogenitaltrakts, Hypercholesterinämie, hormonelles Ungleichgewicht, Hyperglykämie, Hypersalivation, Hypertonie, Hypotonie, Hypothyreose, Infektionskrankheit, entzündliche Erkrankungen, Menopause, Menstruationsregulierung, Migräne, affektive Störungen, Schmerzen des Bewegungsapparats, Übelkeit, Neoplasmen, Adipositas, Parkinson-Krankheit, Lungenerkrankung, Atemwegserkrankung, sexuelle Dysfunktion, Schlafstörung, Raucherentwöhnung, Vaginalerkrankungen, Erbrechen.

15. Vorrichtung nach einem der Ansprüche 1 bis 13 oder Vorrichtung zur Verwendung nach Anspruch 14, wobei die Vorrichtung zur Verabreichung von Arzneimitteln an die Mund-, Nasen- oder Urogenitalschleimhaut dient, wobei die Vorrichtung vorzugsweise ferner einen Träger umfasst, der an die betreffende Körperregion angepasst ist.

## Revendications

1. Dispositif d'administration de médicament pour l'administration de médicament par voie muqueuse et/ou transmuqueuse comprenant au moins un réservoir de médicament, ledit réservoir de médicament comprenant :
- au moins un principe actif pharmaceutique préchargé sur du graphène et/ou ses dérivés, tels que l'oxyde de graphène ;
- un microcontrôleur capable de délivrer un courant électrique à la partie interne dudit réservoir de médicament et capable de se connecter à un dispositif électronique,
dans lequel la libération du principe actif pharmaceutique dudit réservoir de médicament est contrôlée par ledit microcontrôleur, et dans lequel :
ledit réservoir de médicament comprend un premier filtre sur le côté du réservoir de médicament opposé au microcontrôleur, ledit filtre est muni de nanopores permettant le passage du principe actif pharmaceutique mais pas le passage du graphène et/ou de ses dérivés, ou
le graphène et/ou ses dérivés sont fonctionnalisés avec une ou plusieurs nanoparticules magnétiques formant des nanoparticules magnétiques à base de graphène et le microcontrôleur est capable de délivrer un champ nanomagnétique contrôlé par courant empêchant les nanoparticules magnétiques à base de graphène d'atteindre la muqueuse
dans lequel les dérivés de graphène sont choisis dans le groupe constitué par l'oxyde de graphène, l'oxyde de graphène réduit, le graphène hydrogéné, le graphane, le graphone, le graphyne, le graphdiyne, le fluorographène, éventuellement modifiés par fonctionnalisation covalente ou non covalente avec une molécule organique ou inorganique.

2. Dispositif selon la revendication 1, dans lequel ledit ingrédient pharmaceutique est préchargé sur de l'oxyde de graphène, de préférence ledit oxyde de graphène étant sous la forme d'une ou de plusieurs couches.

3. Dispositif selon la revendication 1 ou 2, dans lequel ledit réservoir de médicament comprend en outre un élément électromagnétique et ledit microcontrôleur délivre en outre un champ magnétique contrôlé par courant à la partie interne dudit réservoir de médicament par l'intermédiaire dudit élément électromagnétique.

4. Dispositif selon la revendication 3, dans lequel ledit réservoir de médicament comprend en outre une porte placée à proximité dudit élément électromagnétique et commandée par celui-ci, permettant de commander l'ouverture et la fermeture du réservoir.

5. Dispositif selon l'une quelconque des revendications précédentes, dans lequel ledit réservoir de médicament comprend en outre au moins une électrode et ledit microcontrôleur délivre un courant électrique à la partie interne dudit réservoir de médicament par l'intermédiaire de ladite au moins une électrode.

6. Dispositif selon l'une quelconque des revendications précédentes, dans lequel le microcontrôleur est un système électronique embarqué numérique miniaturisé alimenté par batterie.

7. Dispositif selon l'une quelconque des revendications précédentes, dans lequel ledit réservoir de médicament a au moins un côté configuré pour faire face à la muqueuse d'un sujet et ledit réservoir de médicament comprend en outre un bouchon de réservoir situé à l'extrémité du réservoir de médicament qui est configuré pour faire face à la muqueuse.

8. Dispositif selon l'une quelconque des revendications précédentes, dans lequel ledit dispositif comprend en outre un ou plusieurs compartiments électrolytiques placés à proximité dudit réservoir de médicament et comprenant une solution électrolytique

9. Dispositif selon l'une quelconque des revendications précédentes, dans lequel ledit réservoir de médicament comprend en outre un compartiment d'excipient séparé par le compartiment comprenant le principe actif pharmaceutique chargé sur du graphène et/ou ses dérivés par un second filtre, ledit compartiment d'excipient comprenant un ou plusieurs excipients.

10. Dispositif selon l'une quelconque des revendications précédentes, dans lequel ledit réservoir de médicament comprend en outre un couvercle de filtre placé au-dessus dudit premier filtre, ledit couvercle de filtre étant de préférence flanqué d'une paire d'électrodes permettant de contrôler la durée de filtration du contenu du réservoir.

11. Dispositif selon l'une quelconque des revendications précédentes, dans lequel ledit microcontrôleur est ou comprend un microprocesseur.

12. Dispositif selon l'une quelconque des revendications précédentes, dans lequel le principe actif pharmaceutique est destiné au traitement d'au moins l'un des troubles suivants : troubles gastro-intestinaux, troubles génito-urinaires, troubles psychiatriques, troubles neurologiques et carences nutritionnelles, y compris, mais sans s'y limiter, insuffisance surrénalienne, allergies, troubles anxieux, asthme, trouble déficitaire de l'attention avec hyperactivité, trouble bipolaire, douleurs chroniques et aiguës, bronchite chronique, bronchopneumopathie obstructive chronique, bronchopneumopathie obstructive chronique, congestion, crampes, syndrome de vomissements cycliques, démence, syndrome de dépendance, diabète sucré, épilepsie, fièvre, troubles génito-urinaires, hypercholestérolémie, déséquilibre hormonal, hyperglycémie, hypersalivation, hypertension, hypotension, hypothyroïdie, maladies infectieuses, maladies inflammatoires, ménopause, régulation menstruelle, migraine, troubles de l'humeur, douleurs musculo-squelettiques, nausées, néoplasmes, obésité, maladie de Parkinson, maladies pulmonaires, maladies respiratoires, dysfonctionnements sexuels, troubles du sommeil, sevrage tabagique, maladies vaginales, vomissements.

13. Dispositif selon l'une quelconque des revendications précédentes, dans lequel le principe actif pharmaceutique est choisi dans le groupe constitué par au moins l'un des suivants : acyclovir, albutérol, alclométasone, alfentanil, extraits d'allergènes, alloprégnanolone, almotriptan, aloradine, alprazolam, alprostadil, amoxicilline, amphétamines, androstanediol, apomorphine, arctigénine, articaïne, acide ascorbique, asénapine, atomoxétine, atorvastatine, atropine, azathioprine, azélastine, barbiturique, béclométasone, bisphosphonates, budésonide, bupivacaïne, buprénorphine, bupropion, caféine, cannabidiol, captopril, carfentanil, chlordiazépoxide, chlorophéniramine, chlorpromazine, cholécalciférol, clonidine, clorazépate, acide clorazépique, cobalamine, cortisone, cromoglycate de sodium, cyclobenzaprine, desmopressine, dexaméthasone, dextroamphétamine, diazépam, diéthylcarbamazine, dihydroergotamine, dinoprostone, donépézil, éconazole, élétriptan, endorphines, enképhalines, éphédrine, épinéphrine, ergoloïde, ergonovine, ergotamine, estazolam, estradiol, œstropipate, étonogestrel, fentanyl, fluconazole, fludrocortisone, fluoxétine, flurazépam, fluticasone, framycétine, frovatriptan, furosémide, gabapentine, ganaxolone, gentamicine, glimépiride, glucagon, gramicidine, guaïfénésine, halazépam, héparine, hydrocortisone, hyoscyamine, ibuprofène, insuline ipratropium, isoprotérénol, ivermectine, kétamine, kétoprofène, labétalol, lévothyroxine, lidocaïne, lofentanil, loratadine, lorazépam, magnésium, mélatonine, mépivacaïne, metformine, méthadone, méthylphénidate, méthylsergide, méthyltestostérone, métoclopramide, métoprolol, métronidazole, midazolam, midazolam, misoprostol, mométasone, morphine, nadolol, nalbuphine, naloxone, naltrexone, naproxène, naratriptan, nicotine, nifédipine, nimésulide, nitroglycérine, noréthindrone, novocaïne, olanzapine, ondansétron, opiacés, oxycodone, oxycodone, oxymétazoline, oxymétazoline, oxytocique, paracétamol, pentobarbital, phényléphrine, phytonadione, pilocarpine, prazosine, prednisone, prégabaline, prégnanolone, prilocaïne, prilocaïne, prométhazine, propofol, propranolol, prostaglandine, pyridoxine, rétinol, rizaptril, acide salicylique, salmétérol, scopolamine, ségestérone, sélégiline, sildénafil, sufentanil, sumatriptan, témazépam, terbutaline, tétracaïne, thiamine, thymosine, timolol, tinidazole, tocophérol, tramadol, triamcinolone, triazolam, tryptophane, vardénafil, varénicline, vérapamil, acide zolédronique, zolmitriptan et zolpidem.

14. Dispositif selon la revendication 13 pour une utilisation dans le traitement d'au moins l'un(e) des troubles ou affections suivant(e)s : gastro-intestinaux, troubles génito-urinaires, troubles psychiatriques, troubles neurologiques et carences nutritionnelles, y compris, mais sans s'y limiter, insuffisance surrénalienne, allergies, troubles anxieux, asthme, trouble déficitaire de l'attention avec hyperactivité, trouble bipolaire, douleurs chroniques et aiguës, bronchite chronique, bronchopneumopathie obstructive chronique, bronchopneumopathie obstructive chronique, congestion, crampes, syndrome de vomissements cycliques, démence, syndrome de dépendance, diabète sucré, épilepsie, fièvre, troubles génito-urinaires, hypercholestérolémie, déséquilibre hormonal, hyperglycémie, hypersalivation, hypertension, hypotension, hypothyroïdie, maladies infectieuses, maladies inflammatoires, ménopause, régulation menstruelle, migraine, troubles de l'humeur, douleurs musculo-squelettiques, nausées, néoplasmes, obésité, maladie de Parkinson, maladies pulmonaires, maladies respiratoires, dysfonctionnements sexuels, troubles du sommeil, sevrage tabagique, maladies vaginales, vomissements.

15. Dispositif selon l'une quelconque des revendications 1 à 13, ou dispositif pour une utilisation selon la revendication 14, dans lequel ledit dispositif est destiné à l'administration de médicaments à la muqueuse buccale, nasale ou urogénitale, de préférence ledit dispositif comprenant en outre un support adapté à la région corporelle d'intérêt.
